# EUROPEAN PATENT APPLICATION

(11) **EP 2 420 571 A1**
(43) Date of publication of application: **22.02.2012**
(21) Application number: 10731288.6
(22) Date of filing: 15.01.2010
(51) Int. Cl.: C12N 15/09, C12N 1/15, C12N 1/19, C12N 1/21, C12N 5/10, C12P 21/02

(54) **HIGH EXPRESSION PROMOTER AND METHOD FOR PRODUCING GENE PRODUCT USING SAME**

(30) Priority: 16.01.2009 JP 2009007950
(71) Applicant: Otsuka Chemical Co., Ltd., Osaka-shi, Osaka 540-0021 (JP)
(72) Inventor: SAKURAI, Jun, Tokushima-shi Tokushima 771-4263 (JP); NAGAHAMA, Masahiro, Tokushima-shi Tokushima 771-1154 (JP); ODA, Masataka, Tokushima-shi Tokushima 770-0944 (JP)
(74) Representative: Flaccus, Rolf-Dieter
(86) International application number: PCT/JP2010/050387
(87) International publication number: WO 2010/082619

(57) **Abstract**

Disclosed is a promoter which enables the expression of a gene product in a large quantity in Bacillus subtilis. Also disclosed is a method for producing a gene product using the promoter. Specifically disclosed are: a nucleic acid molecule which contains a promoter region derived from a toxin gene of a bacterium belonging to the genus Clostridium and can enhance the expression of a heterologous gene operably linked to the nucleic acid molecule; a nucleic acid construct which contains the nucleic acid molecule and the heterologous gene; a vector carrying the nucleic acid construct; a host cell which is transformed with the vector; and a method and a kit for producing an expression product of the heterologous gene using the nucleic acid molecule, the nucleic acid construct, the vector or the host cell.

## Description

### [TECHNICAL FIELD]

The present invention relates to a high-expression promoter and a process for producing a gene product using said promoter. Specifically, the invention relates to a nucleic acid molecule comprising a promoter region derived from a toxin gene of *Clostridium* bacterium, a nucleic acid construct comprising said nucleic acid molecule and a heterologous gene operably linked thereto, a vector comprising them, a host cell transformed with said vector, and a process and a kit for the production of a gene product using the same.

### [BACKGROUND ARTS]

Progresses in the genetic engineering have widely enabled us to introduce and express a desired gene in a microorganism such as bacteria and yeasts and a cultured cell of a higher animal such as a mammal, to produce various gene products such as proteins. Prevailing among such production method is a procedure using *Escherichia coli* (*E. coil*)*,* for their ease in culturing operation, high reproductivity and high level of gene expression, and such procedure is widely used in industry. However, a gene product that has been expressed in a Gram-negative bacterium *E. coli is* accumulated in the periplasm between the inner and outer membranes. Therefore, in order to extract the gene product it is necessary to first destroy the cell body before separating and purifying the product of interest. Further, since LPSs (lipopolysaccharides), which exist on the outer membrane of *E. coli,* act as pyrogens in a living organism such as human, it is necessary to remove such pyrogens when a gene product produced employing *E. coli* is to be used, requiring a considerable time and cost for the purification of the gene product.

A Gram-positive bacterium *Bacillus subtilis* (*B. subtilis*) has been drawling attention as a bacterial species having no such disadvantages. *B. subtilis* does not possess a pyrogen as above, does not have a pathogenicity, and in many cases secretes its gene product to the outside of the cell body. It is therefore useful for the production of a gene product which is to be applied to a living organism as, for example, a medicament or food. However, *B. subtilis* has a disadvantage that the level of its production of a gene product is relatively low compared to E. *coli,* which hinders the industrial use of this bacterial species. One reason for this low-level production of a gene product by *B. subtilis* is the absence of a promoter with good operability and expression efficacy like *lac* promoter of *E. coli.* Given such circumstances, there have been attempts to search for a promoter that effectively operates in *B. subtilis.*

For instance, Patent Literature 1 describes a promoter containing a particular sequence; Patent Literature 2 describes a hybrid promoter containing a promoter of the alpha-amylase gene of *Bacillus amyloliquefaciens* and an enhancer receptor such as those originate from the alkaline protease gene of *B. subtilis*; Patent Literature 3 describes a DNA fragment which comprises an upstream region of alkaline cellulase K-64 and which may increase the expression of structural genes attached to the downstream thereof; Patent Literature 4 describes a promoter which is derived from a variant of the alpha-amylase gene promoter of *Bacillus licheniformis*; Patent Literature 5 describes promoters comprising an altered AmyQ promoter sequence, cryIIIA mRNA stabilizing sequence and a sequence in which these sequences are arranged in tandem; Patent Literature 6 describes an altered promoter in which a special sequence has been introduced near the 3' terminal of the alpha-amylase gene promoter of *Bacillus amyloliquefaciens*; Patent Literature 7 describes promoters derived from the upstream regions of several genes which are expressed in a stationary phase-specific manner in *B, subtilis*; and Non-Patent Literature 1 describes a promoter derived from the MWP gene of *Bacillus brevis.*
However, none of the above-mentioned promoters has yielded a satisfactory result, leaving a need for further development of promoters.

### [PRIOR ART LITERATURES]

### [PATENT LITERATURES]

Patent Literature 1 : JP A 60-137291
Patent Literature 2 : JP A 6-500689
Patent Literature 3 : JP A 6-217781
Patent Literature 4 : JP A 7-504085
Patent Literature 5 : JP A 2002-504379
Patent Literature 6 : JP A 2002-272466
Patent Literature 7 : WO 2002/072819

### [NON-PATENT LITERATURES]

Non-Patent Literature 1 : Yamagata et al., Proc Natl Acad Sci U S A. 1989 May;86(10):3589-93

### [SUMMARY OF THE INVENTION]

### (PROBLEMS TO BE SOLVED BY THE INVENTION]

An object of the present invention is to provide a promoter that is capable of a high level expression of a gene product in *B. subtilis,* and a process for the production of a gene product utilizing said promoter.

### [MEANS FOR SOLVING THE PROBLEMS]

The inventors carried on an intensive research in order to solve the problems above, and found that the expression of a gene product was dramatically increased by linking an upstream sequence of a toxin gene of *Clostridium* bacterium, especially the iota (τ)-toxin of *Clostridium perfringens* (*C. perfringens*)*,* to a heterologous gene and expressing it in *B. subtilis,* thereby completing the invention. Namely, the invention relates to the followings:
(1) A nucleic acid molecule comprising a promoter region derived from a toxin gene of a *Clostridium* bacterium, which enhances the expression of a heterologous gene operably.linked thereto.
(2) The nucleic acid molecule of (1), wherein the toxin gene is selected from the group consisting of *C. perfringens* alpha (α)-toxin, epsilon (ε)-toxin and iota (τ)-toxin, botulinum toxin, and tetanus toxin.
(3) A nucleic acid construct comprising the nucleic acid molecule of (1) or (2) above and a heterologous gene operably linked thereto.
(4) A vector comprising the nucleic acid molecule of (1) or (2) above or the nucleic acid construct of (3) above.
(5) A host cell that has been transformed with the vector of (4) above.
(6) A process for the production of a gene product, comprising the step of culturing the host cell of (5) above.
(7) A kit for the production of a gene product, comprising the nucleic acid molecule of (1) or (2) above, the nucleic acid construct of (3) above, the vector of (4) above, and/or the host cell of (5) above.

### [THE EFFECTS OF THE INVENTION]

By the present invention, the levels of expression of various gene products can be remarkably increased in *B. subtilis,* which had been considered to be difficult to be used for a high-level expression of a gene product. Therefore, the invention allows obtaining a large amount of a pyrogen-free, biologically-safe gene product by a simple purification operation, being expected to provide a great contribution in the fields of medical and food products, in particular.
In addition, the kit for the production of a gene product of the present invention is capable of producing various types of gene product readily and in large amount, and therefore can advantageously be utilized in a research institute where many types of small lots of gene product are required to be expressed.

### [BRIEF DESCRIPTION OF THE DRAWINGS]

[Fig. 1] Fig. 1 is a schematic diagram showing the location of *C. perfringens τ*-toxin promoter (black arrow) in pCIP.
[Fig. 2] Fig. 2 is a diagram showing the sequence of the region of pCIP where *C. perfringens* τ-toxin promoter has been inserted. In the figure, the shaded bold part shows the inserted promoter region and the underlined parts show the restriction sites, respectively.

### [DESCRIPTION OF EMBODIMENTS]

Unless otherwise stated herein, the scientific and technical terms used in the context of the present invention have meanings which are normally understood by those having an ordinary skill in the art. In general, the terms and the techniques used in the context of the cell and cell culture, molecular biology, microbiology, genetics, protein and nucleic acid chemistry, and hybridization described herein are those which are well known and normally used in the art. Unless specifically stated, the methods and the techniques of the present invention are performed according to the routine methods well known in the art, as described in various references cited herein. Such references include, for example, Sambrook et al., Molecular Cloning: A Laboratory Manual, 2d ed., Cold Spring Harbor Laboratory Press (1989) and Sambrook et al., Molecular Cloning: A Laboratory Manual, 3d ed., Cold Spring Harbor Press (2001), Ausubel et al., Current Protocols in Molecular Biology, Greene Publishing Associates (1992, and 2000 suppl.), Ausubel et al., Short Protocols in Molecular Biology: A Compendium of Methods from Current Protocols in Molecular Biology - 4th Ed., Wiley & Sons (1999), Harlow and Lane, Antibodies: A Laboratory Manual, Cold Spring Harbor Laboratory Press (1990), and Harlow and Lane, Using Antibodies: A Laboratory Manual, Cold Spring Harbor Laboratory Press (1999).

The enzymatic reactions and purification techniques are to be performed in accordance with the instruction of the manufacturer, as normally carried out in the art or as described herein. The terms, experimental procedures and techniques used in the context of the analytical chemistry and synthetic organic chemistry described herein are those which are well known and normally used in the art. Also, the standard techniques in the art are to be used in genetic engineering, cell culturing, chemical synthesis and chemical analysis, etc.
An aspect of the present invention relates to a nucleic acid molecule comprising a promoter region derived from a toxin gene of a *Clostridium* bacterium, which enhances the expression of a heterologous gene operably linked thereto.
The *Clostridium* bacterium in the present invention is not particularly limited as long as it has a toxin gene, and includes, for example, *Clostridium perfringens* (*C. perfringens*), *Clostridium botulinum* (*C. botulinum*), *Clostridium tetani* (*C. tetani*), *C. novyi, C. septicum, C. histolycum, C. difficile,* etc.

The toxin gene of the *Clostridium* bacterium in the present invention is not particularly limited as long as it comprises in its upstream region a sequence having a promoter activity, and includes, for example, *C. perfringens* α-toxin gene (see, e.g., Titball et al., Infect Immun. 1989 Feb; 57(2):367-76), ε-toxin gene (see, e.g., Hunter et al., Infect Immun. 1992 Jan; 60(1): 102-10) and τ-toxin gene (see, e.g., Perelle et al., Infect Immun. 1993 Dec; 61(12): 5147-56), botulinum toxin gene (see, e.g., Whelan et al., Eur J Biochem. 1992 Mar 1; 204(2): 657-67, Thompson et al., Eur J Biochem. 1990 Apr 20; 189(1): 73-81), tetanus toxin gene (see, e.g., Eisel et al., EMBO J. 1986 Oct; 5(10): 2495-502), etc. The nucleic acid sequences of these genes are known and available from the references above and the genetic databases such as NCBI. For instance, the gene sequences of *C. perfringens* α-toxin*,* ε-toxin and τ-toxin have been registered at and freely available from GenBank by Accession Nos. X13608, M80837 and X73562; the gene sequences of types A to F botulinum toxins by X52066, X78229, S74768, S49407, X62683 and X71086; and the gene sequence of the tetanus toxin by X04436, respectively.

In the present invention, a promoter region means a gene region present in the upstream of a coding region (structural gene) of a gene, e.g., in 5' non-coding region, and whose presence allows initiating the transcription of the structural gene. A promoter region typically contains a -10 region (also known as pribnow box) which is located at about 10 bases upstream (-10) to the transcription initiation site (+1), and has a motif such as 5'-TATAAT-3' or its analogous sequence and/or a -35 region located at about 35 bases upstream (-35) and having a motif such as 5'-TTGACA-3' or its analogous sequence. Thus, in one embodiment of the present invention, a promoter region includes a 5' non-coding region of a gene selected from the group consisting of *C. perfringens* α-toxin gene, ε-toxin gene and τ-toxin gene, botulinum types A and E toxin genes and tetanus toxin gene, more typically, a sequence according to SEQ ID NOs: 1 to 6 or a partial sequence thereof.

Preferred partial sequence is, or comprises, a -35 region and/or a -10 region, namely, positions 681 to 709 of SEQ ID NO: 1 (SEQ ID NO: 7), positions 97 to 125 of SEQ ID NO: 2 (SEQ ID NO: 8), positions 85 to 114 of SEQ ID NO: 3 (SEQ ID NO: 9), positions 19 to 46 of SEQ ID NO: 4 (SEQ ID NO: 10), positions 103 to 108 of SEQ ID NO: 5 (SEQ ID NO: 11) or positions 193 to 200 of SEQ ID NO: 6 (SEQ ID NO: 12). A sequence comprising a -35 region and/or -10 region includes for example, without limitation, a portion from a -35 region or -10 region to the start codon, which is, for each of the above-mentioned toxin gene, specifically, a sequence of SEQ ID NOs: 13 to 18. The portion to be selected as the partial sequence may be appropriately determined in consideration of various conditions such as the level of the promoter activity or the presence of an appropriate restriction site. The level of the promoter activity may be assessed, for example, by comparing the levels of gene expressions which are operably linked to the subject partial sequence, as described below. Also, a restriction site on a nucleic acid sequence can readily be determined using tools for genetic analysis available on Internet, such as Webcutter (http://www.firstmarket.com/cutter/cut2.htm1) or NEBcutter (http://tools.neb.com/NEBcutter2/index.php). In case where no desired restriction site is present, a restriction site may be added into the partial sequence by site-specific mutagenesis, etc., as described hereinbelow.

On the other hand, a nucleic acid molecule which does not have a typical motif as above, but still exists in the upstream of the structural gene of a *Clostridium* toxin gene and is capable of initiating the transcription of the gene is also encompassed in the promoter of the present invention. A promoter region may be identified based on the presence of a typical motif as above; or all or a part of the 5' non-coding region of a gene may conveniently be taken as a promoter region. Since it appears that RNA polymerase, which is involved in mRNA synthesis, binds to a promoter region, it is also possible to identify a promoter region based on the sequence recognized by RNA polymerase. The part of the gene which would actually function as a promoter may be identified, for example, by mutating (e.g., partially deleting or substituting) the upstream region of the coding region and assessing the change in the gene expression. When the gene expression is decreased by the mutation, the mutated part is the promoter region, or includes the promoter region, or partially overlaps with the promoter region.

The nucleic acid molecule of the present invention may also comprise a nucleic acid molecule having a nucleic acid sequence analogous to the promoter region of a naturally occurring *Clostridium* toxin gene and having an activity equivalent to that of said region, e.g., a transcription promoting activity (a promoter activity). The above-mentioned "analogous nucleic acid sequence" includes, for example, a nucleic acid sequence having 60% or greater, 65% or greater, 70% or greater, 75% or greater, 80% or greater, 85% or greater, 90% or greater, 95% or greater, 98% or greater, or 99% or greater homology to the nucleic acid sequence of a naturally occurring promoter region (e.g., a nucleic acid sequence according to SEQ ID NOs: 1 to 6); or a nucleic acid sequence of a nucleic acid molecule which hybridizes to a naturally occurring promoter region (e.g., a nucleic acid molecule encoded by a nucleic acid sequence according to SEQ ID NOs: 1 to 6) under stringent conditions.
Herein, a homology is a term well known in the art, and refers, for example, to the proportion of bases that match between multiple nucleic acid sequences when they are appropriately aligned. A homology of nucleic acid sequences may be determined utilizing tools publicly available on Internet, e.g., BLAST (http://blast.ncbi.nlm.nih.gov/Blast.cgi) or various commercial software.

The term "stringent condition" used herein is a well known parameter in the art and described in standard protocols such as Sambrook et al., Molecular Cloning: A Laboratory Manual, 3d ed., Cold Spring Harbor Press (2001) and Ausubel et al., Current Protocols in Molecular Biology, Greene Publishing Associates (1992).
A stringent condition in the present invention refers to a hybridization at 65°C in a hybridization buffer comprised of 3.5 x SSC (0.15M sodium chloride /0. 15M sodium citrate , pH 7), Ficoll 0.02%, polyvinylpyrrolidone 0.02%, bovine serum albumin 0.02%, NaH₂PO₄ 25 mM (pH 7), SDS 0.05% and EDTA 2 mM. After hybridization, the membrane to which DNA was transferred is washed in 2 x SSC at room temperature, then in 0.1 to 0.5 x SSC/0.1 x SDS at a temperature up to 68°C. Alternatively, a stringent hybridization may be performed using a commercially available hybridization buffer such as ExpressHyb™ Hybridization Solution (Clontech), under the conditions for hybridization and washing described by the manufacturer.
There will be other conditions and reagents which may be used to give a similar level of stringency, though these are not described in particular herein since a person with an ordinary skill in the art should be familiar with such conditions. However, a condition may be appropriately adjusted in order to allow an unambiguous identification of a sequence analogous to a promoter region of a *Clostridium* toxin gene.

A promoter region in the present invention may be one included in a naturally occurring *Clostridium* toxin gene or may be a variant of a naturally occurring promoter region with a mutation. A mutation includes a deletion, substitution or addition of any nucleotide sequence, e.g., an alteration other than in the -10 region and/or the -35 region, an alteration in a part from the -10 region to the -35 region (including a part between both regions), an alteration of a motif in the -10 region and/or the -35 region; an increase/decrease in the number of nucleotide and/or substitution of nucleotides between these regions, and an increase/decrease in the number of nucleotide and/or substitution of nucleotides between either of these regions and the transcription initiation site.
In one embodiment of the present invention, a preferred variant of a nucleic acid molecule is a nucleic acid molecule which comprises one or more mutations in the nucleic acid sequence of SEQ ID NO: 1 and comprises the nucleic acid sequence of SEQ ID NO: 7; a nucleic acid molecule which comprises one or more mutations in the nucleic acid sequence of SEQ ID NO: 2 and comprises the nucleic acid sequence of SEQ ID NO: 8; a nucleic acid molecule which comprises one or more mutations in the nucleic acid sequence of SEQ ID NO: 3 and comprises the nucleic acid sequence of SEQ ID NO: 9; a nucleic acid molecule which comprises one or more mutations in the nucleic acid sequence of SEQ ID NO: 4 and comprises the nucleic acid sequence of SEQ ID NO: 10; a nucleic acid molecule which comprises one or more mutations in the nucleic acid sequence of SEQ ID NO: 5 and comprises the nucleic acid sequence of SEQ ID NO: 11; or a nucleic acid molecule which comprises one or more mutations in the nucleic acid sequence of SEQ ID NO: 6 and comprises the nucleic acid sequence of SEQ ID NO: 12.

The number of the nucleotide to be mutated is not particularly limited, though it may be in the range of one to several, 1 to 10, 1 to 20, 1 to 30, 1 to 50, or 1 to 100.
Among the variants described above, a mutation that elevates the promoter activity, namely, a mutation that increases the expression of a gene of the interest, is preferred. Moreover, a desired restriction site may be added to a promoter region in the present invention for the convenience in cloning. The method for introducing a restriction site into a promoter region is not particularly limited and may be performed using any known procedures such as, for example, various methods for site-specific mutagenesis such as the inverse PCR, overlap extension PCR and megaprimer PCR, and a method of amplification, e.g., by PCR, using a primer to which a restriction site has been added.
The nucleic acid molecule of the present invention is typically a DNA, although it may be, in some cases, an RNA or an artificial nucleic acid such as PNA, LNA or various modified nucleic acids (such as phosphorothioate modified nucleic acid).

A heterologous gene in the present invention means a gene which is different from a gene from which the promoter region included in the nucleic acid molecule of the present invention has been derived. A species of the organism may be the same or different. For instance, if the nucleic acid molecule of the present invention derives from *C. perfringens* τ-toxin gene, a heterologous gene may be any gene other than *C. perfringens τ*-toxin gene, e.g., without limitation, *C. perfringens* α-toxin gene, ε-toxin gene, botulinum toxin gene or tetanus toxin gene.
A preferred heterologous gene in the present invention includes, without limitation, e.g., a gene derived from bacteria, preferably Gram-positive bacteria (such as C. *perfringens* α-toxin, ε-toxin and τ-toxin, botulinum toxin and tetanus toxin), a gene of a low molecular weight protein (e.g., those of a molecular weight at or less than 100 kDa, especially a molecular weight at or less than 50 kDa), a gene of a protein with a drug action (such as an enzyme, hormone, antibody and cytokine originates from the subject to be treated, such as a human or non-human animal). A heterologous gene of the present invention typically encodes for a protein, thought it may encode for a nucleic acid molecule such as an antisense nucleic acid or an siRNA..
A heterologous gene may comprise only a coding region, or may comprise a non-coding region. A non-coding region may also comprise various functional sequences such as a promoter, an enhancer, a terminator or a ribosome-binding sequence.

A heterologous gene may also comprise various alterations that are advantageous for the expression, secretion and/or purification of a gene product. Such alteration includes, without limitation, e.g., an addition of a secretory signal sequence, an addition of a tag sequence for purification (such as His tag, GST tag, S tag, T7 tag).
A secretory signal includes, without limitation, e.g., those derived from acetolactate decarboxylase, alkaline cellulase, alkaline phosphatase, alkaline protease, amylase (such as α-amylase), bacillopeptidase, chitinase, cyclodextrin glucanotransferase, β-glucanase, β-lactamase, levanase, levansucrase, β-mannanase, metalloprotease, MWP (middle wall protein), neutral protease, OWP (outer wall protein), RNase, sphingomyelinase, subtilisin and xylanase of a *Bacillus* bacterium (for specific sequence, see, e.g., Microbiol Rev. 1993 Mar;57 (1):109-37).

In the present invention, by a heterologous gene being "operably linked" to a nucleic acid molecule of the present invention, it is meant that the heterologous gene and the nucleic acid molecule of the present invention are chemically bound such that, when a nucleic acid construct of the present invention comprising the nucleic acid molecule of the present invention and the heterologous gene operably linked thereto is introduced into an appropriate host cell, said heterologous gene will be expressed in a normal condition in that host cell. A normal condition refers to, in a host cell, a state in which there has been no treatment which would enhance or decrease the expression of said heterologous gene, or a state in which the cell is not suffering from a pathogen which would enhance or decrease the expression of said heterologous gene. As long as the above condition is satisfied, a nucleic acid molecule of the present invention may be linked to either the upstream or downstream of the heterologous gene, though it typically is located to the upstream of the heterologous gene. Here, the upstream and downstream of a heterologous gene means the 5' side and 3' side of the heterologous gene, respectively. The nucleic acid molecule of the present invention may be directly linked to a heterologous gene, or may be linked via an intervening nucleotide sequence (a spacer). The positional relationship between a nucleic acid molecule of the present invention and a heterologous gene is preferably identical to or similar to the positional relationship of a promoter region contained in the nucleic acid molecule of the present invention and the coding region of the gene from which the promoter is derived from, though it is not limited as long as the conditions above are fulfilled.

In the present invention, by "linking" a nucleic acid molecule of the present invention and a heterologous gene, it is meant that the nucleic acid molecule of the present invention and the heterologous gene are chemically bound either directly or via a spacer. In the present invention, when the nucleic acid molecule is a DNA or an RNA, such a chemical binding typically includes a phosphodiester linkage, though it may be other linkage such as phosphorothioate linkage as long as it is operable.
In the present invention, by a nucleic acid molecule of the present invention will "enhance the expression" of a heterologous gene, it is meant that, by operably linking the heterologous gene to the nucleic acid molecule of the present invention, the expression of the heterologous gene in an appropriate host cell will be elevated compared to when it is not linked to the nucleic acid molecule of the present invention. The elevation of the expression may be assessed as an increase in the expression level per unit time, and/or as a shortening of the period to obtain a given amount of expression product. In the present invention, a level of expression is higher than when the nucleic acid molecule of the present invention is not linked, preferably 1.5-fold or higher, more preferably 2-fold or higher, further preferably 2.5-fold or higher, yet more preferably 3-fold or higher, particularly preferably 4-fold or higher. The enhancing effect of the nucleic acid molecule of the present invention on the expression of a heterologous gene may vary depending on the type of the heterologous gene to be linked.

The amount of the expression product may be assessed by culturing for a given time period a host cell in which the nucleic acid construct of the present invention has been introduced, and measuring the amount of the expression product within the culture supernatant or within the host cell lysate using known procedures. Such procedures include, without limitation, e.g., general methods for protein measurement including methods using antibodies such as EIA, ELISA, IRA, IRMA and Western blotting, ultraviolet absorption method, colorimetric methods such as Bradford method (Coomassie blue method), Lowry method (phenol reagent method), BCA method (bicinchoninic acid method), quantification by the comparison of electrophoresis patterns, as well as various other procedures based on the nature of the expression product of the heterologous gene including, e.g., when the expression product is an enzyme, quantification of the reaction with its substrate, or, when the expression product is a substrate of an enzyme, quantification of the reaction with its enzyme, or, when the expression product is a physiologically active substance, quantification of the physiological c reaction.
A level of expression may also readily be compared using as a heterologous gene a reporter gene which is easy to be detected, such as CAT (chloramphenicol acetyltransferase), DsRed (Discosoma sp. Red Fluorescent Protein), Green Fluorescent Protein (GFP), β-glucuronidase (GUS), lacZ and luciferase.

The nucleic acid molecule of the present invention may comprise two or more promoter regions. In this case, at least one of the promoter regions is that derived from *Clostridium* toxin gene. Accordingly, the nucleic acid molecule of the present invention may comprise at least one promoter region which is derived from *Clostridium* toxin gene, and at least one promoter region which is not derived from *Clostridium* toxin gene. Two or more promoter regions may be linked to each other either directly or via intervening sequence.
The nucleic acid molecule of the present invention may also comprise a ribosome binding site such as Shine-Dalgarno (SD) sequence which is involved in translation. SD sequence is a purine base-rich, 3 to 9-base-long sequence having a motif such as 5'-AGGAGG-3' or its analogous sequence, and is considered to be bound by 16SrRNA. A ribosome binding site is preferably contained in downstream of the transcription initiation site. A nucleic acid molecule of the present invention comprising SD sequence includes, for example, those comprising the sequences of SEQ ID NOs: 1 to 6, or the partial sequences thereof, i.e., the sequences described in SEQ ID NOs: 19 to 24.

The present invention also relates to a nucleic acid construct comprising a nucleic acid molecule of the present invention and a heterologous gene operably linked thereto. The nucleic acid construct of the present invention may have, besides the nucleic acid molecule of the present invention and the heterologous gene, a terminator that terminates the transcription (also referred to as a transcription termination signal). A terminator may be a part of the heterologous gene, or may be derived therefrom, or may not be derived therefrom. General examples of a terminator are well known to those skilled in the art, and any of these may be used. Specifically, it includes, without limitation, e.g., a sequence having a palindromic structure which renders the transcribed mRNA to have a strong hairpin structure (e.g., a GC-rich sequence). The nucleic acid construct of the present invention may also include, besides those aforementioned, other gene sequences which allow an efficient replication and expression of the construct within desired cell.

The nucleic acid construct of the present invention may be introduced into a host cell by various nucleic acid introducing methods, such as calcium phosphate method, lipofection method, ultrasound transfection method, electroporation method, particle gun method, microinjection method, liposome method (e.g., by cationic liposome), competent cell method, protoplast method, to allow the expression of its carrying heterologous gene. Furthermore, the nucleic acid construct of the present invention may be incorporated into various expression vectors, which is then used to transfect a host cell to allow the expression of its carrying heterologous gene.

The present invention also relates to a vector comprising a nucleic acid molecule or a nucleic acid construct of the present invention. In the present invention, a vector means any nucleic acid molecule that is capable of incorporating a nucleic acid molecule or nucleic acid construct of the present invention into itself, maintaining it, and introducing it into a host cell gene, amplifying and/or expressing a heterologous gene. A vector typically is constructed by a DNA, though a vector constructed by an RNA may also be used. A vector includes, without limitation, e.g., a plasmid, cosmid, phage, virus, YAC and BAC. Although a vector may be categorized as, according to its use, such as a cloning vector which is to be used for gene cloning, or as an expression vector to be used for gene expression, the vector of the present invention encompasses vectors of these various uses. A vector may include, according to its use, besides the nucleic acid molecule or nucleic acid construct of the present invention, various functional nucleotide sequences useful for, e.g., the incorporation of the nucleic acid molecule or nucleic acid construct, for introduction into a host cell, for replication and for heterologous gene expression. Such nucleotide sequences include, for example, without limitation, a restriction site (such as multicloning site), a replication origin sequence, a selection marker gene sequence, a reporter gene sequence, a promoter sequence and a terminator sequence. Such functional nucleotide sequences are well known to those skilled in the art, and any of the known functional sequences may be used in an appropriate location and orientation.

A vector of the present invention comprises at least a nucleic acid molecule of the present invention. One embodiment of the vector of the present invention therefore does not comprise any subject heterologous gene. Here, a subject heterologous gene does not comprise any functional gene that render an additional function to the vector, e.g., a selection marker gene or a reporter gene. Accordingly, the above-mentioned embodiment of the vector of the invention does not comprise any subject heterologous gene, though it may comprise such functional genes. In this embodiment, the vector preferably has a restriction site, more preferably multicloning site, which is capable of operably linking the nucleic acid molecule of the present invention and the subject heterologous gene, so that any desired heterologous gene can be incorporated therein. Accordingly, a preferred vector of this embodiment may be used for the expression of any heterologous gene. A particularly preferred example of this embodiment includes, without limitation, a vector having the nucleic acid sequence of SEQ ID NO: 33.
In another embodiment, the vector of the present invention comprises a nucleic acid molecule of the present invention and a heterologous gene operably linked thereto, i.e., a nucleic acid construct of the present invention. A particularly preferred example of this embodiment includes, without limitation, a vector having the nucleic acid sequence of any one of SEQ ID NOs: 30, 58 to 62.

A vector may be generated by combining desired functional nucleotide sequences, or may be generated based on various commercially available vectors. Vectors corresponding to various host cells and for various usages are available. Vectors for *Bacillus subtilis* including, for example, pA-spac, pAM1, pAX01, pBS72, pC194, pDG1661, pDG1662, pDG1663, pDG1664, pDG1728, pDG1729, pDG1731, pDG271, pDL, pDK, pDH32, pE194, pEl94-cop6, pGlt-Cm, pGlt-Kan, pHCM02, pHCM04, pHCM05, pHY500, pHY700, pHY4831, pHT110R2L5, pHT210, pHV1431, pHV1432, pIP404, pIP501, pLS20, pLS32, pMLK83, pMTLBS72, pNDH33, pNH200, pNH300, pNH400, pNU100, pNU200, pNU211, pNU211R2L5, pPyr-Cm, pPyr-Kan, pSac-Cm, pSac-Kan, pSM19035, pT127, pT181, pTA1015, pTA1060, pTB19, pTRKH2, pUB110, φ105 and SPβ; vectors for *E. coli* including, for example, pACY177, pACYC184, pBR322, pBluesript, pET, pUC18, pUC19, λgt10 and λgt11; vectors which can be used for both *B. subtilis* and *E. coli (such* as a shuttle vector) including, for example, pBE20, pBE60, pE18, pEB10, pHB201, pHP13, pHPS9, pHV14, pHY300PLK, pLB5, pRB373, pUB18, pUB19 and pWB980 are known (see, e.g., Schumann, Adv Appl Microbiol. 2007;62:137-89). Accordingly, one embodiment of the present invention is a vector in which a nucleic acid molecule or nucleic acid construct of the present invention has been incorporated into one of such vector or its modified version.

The incorporation of a nucleic acid molecule or nucleic acid construct of the present invention into a vector is typically carried out with a restriction enzyme. As a restriction enzyme, any known enzyme known to those skilled in the art such as AatII, AccI, ApaI, AorI, BamHI, BgIII, BsaAI, BsmI, BssHII, BstXI, Cfr9I, ClaI, DdcI, DpnI, DraI, EcoRI, EcoRV, EcoT22I, FokI, FspI, HaeIII, HapI, HapII, HincII, HinfI, HindIII, KpnI, MaeIII, MboI, MluI, MseI, MvaI, Nael, NcoI, NdeI, NheI, NotI, PmaCI, PstI, PvuII, RsaI, SacI, SacII, SaII, Sau3AI, Sau96I, SeaI, SfiI, SmaI, SpeI, SphI, SpII, SspI, TaqI, XbaI, XmnI and XhoI may be used.

The present invention also relates to a host cell transformed with a nucleic acid molecule, nucleic acid construct or vector of the present invention. The host cell in the present invention is not particularly limited as long as a nucleic acid molecule of the present invention can function within that host cell, namely, a heterologous gene that is operably linked to the nucleic acid molecule of the present invention can be expressed within it, and/or, a vector of the present invention can be maintained or replicated within it, and encompasses various cells including prokaryotic cells and eukaryotic cells. In one embodiment of the present invention, a host cell is a prokaryotic cell, typically a bacterial cell. In the present invention, a preferred bacterial species includes without limitation, e.g., a *Bacillus* bacterium such as *Bacillus subtilis* (*B. subtilis*), *Bacillus brevis, Bacillus licheniformis, Bacillus megaterium, Bacillus stearothermophilus,* and *E. coli.* Among these, a *Bacillus* bacterium is preferred for the ease of purifying the expression product and a low risk of contamination of biologically adverse by-product derived from the production steps. *Bacillus subtilis* is particularly preferred. As for *Bacillus subtilis,* various bacterial strains, e.g., BD170 strain, 168 strain, ISW1214 strain, MI114 strain are available. Also, a bacterial strain in which the production of extracellularly secreted protease has been suppressed (such as e.g., 106HL strain, DY-16 strain) may be used.

Transforming a host cell typically refers to introducing an exogenous nucleic acid molecule into a host cell to change the genetic characteristics of the cell, which is carried out, specifically, by introducing a nucleic acid molecule, nucleic acid construct or vector of the present invention into a host cell. Herein, such introduced host cell may especially be referred to as a transformant. For introduction, various methods for introducing a nucleic acid may be used, such as calcium phosphate method, lipofection method, ultrasound transfection method, electroporation method, particle gun method, microinjection method, liposome method (e.g., by a cationic liposome), competent cell method and protoplast method. In the case of a nucleic acid molecule or nucleic acid construct, introduction methods utilizing a virus vector such as, e.g., an adenovirus vector or a retrovirus vector may also be used besides the above-mentioned methods. Introduced nucleic acid molecule, nucleic acid construct or vector will be incorporated into the genome of the host cell, which may allow the expression of the subject heterologous gene, either constitutively or inducibly. When a nucleic acid molecule is to be introduced, it is necessary to be incorporated into the host cell genome such that it will be operably linked to the subjected heterologous gene. Moreover, when a vector is to be introduced, the heterologous gene may be expressed within the host cell without being incorporated into the host cell genome.

A host cell may also be used for maintaining or amplifying a vector of the present invention. For such use, the vector is maintained within the host cell, being either independent of or incorporated into the host cell genome, or is autonomously amplified. In this case, the expression of the heterologous gene may or may not be taken place. In another embodiment, the vector is maintained without being replicated within the same host cell, but it will be replicated upon the division of the host cell. The vector thus maintained or amplified may then be introduced into a host cell for expression.

The present invention also relates to a process for the production of a gene product, comprising a step of culturing a host cell of the present invention (i.e., a transformant). A transformant of the present invention may be cultured by known procedures suitable for each transformant. Culturing is carried out typically in a medium. A medium includes those in various forms such as a solid, semi-solid or liquid, though a liquid medium is preferred for the ease of treating and capability of large-scale culture. Furthermore, a medium to be used may be either a natural or artificial medium, as long as it contains a carbon source, a nitrogen source, inorganic salts, etc. which will be assimilated by the transformant and is capable of an efficient culture. A carbon source may be any one of those which will be assimilated by the transformant, and glucose, fructose, sucrose, maltose, cellobiose, a syrup containing them, a carbohydrate such as starch or starch hydrolysate, an organic acid such as acetic acid or propionic acid, alcohols such as ethanol or propanol, etc. may be used. As a nitrogen source, ammonium, ammonium chloride, an ammonium salt of inorganic or organic acid, such as ammonium sulfate, ammonium acetate, ammonium phosphate, and other nitrogen-containing compounds, as well as a peptone, meat extract, fish extract, yeast extract, corn steep liquor, casein hydrolysate, soymeal and soymeal hydrolysate, various fermentative bacteria and their digested materials, etc., may be used. As inorganic salts, potassium dihydrogen phosphate, dipotassium hydrogenphosphate, magnesium phosphate, magnesium sulfate, sodium chloride, ferrous sulphate, manganese sulfate, copper sulfate, calcium carbonate, etc. may be used.

Culturing of a transformant which utilizes as a host a prokaryote such as *B. subtilis* or *E. coli* or an eukaryote such as an yeast is carried out under an aerobic condition such as in a normal shaking culture or in a deep aerated and agitated culture. The culturing temperature is typically 15 to 50°C, preferably 20 to 40°C, and the culturing period is normally 16 hours to 7 days. The pH in the culture is maintained from 3.0 to 9.0. The adjustment of pH is done using such as an inorganic or organic acid, alkaline solution, urea, calcium carbonate, and ammonium. Moreover, an antibiotic such as ampicillin and tetracycline may be added into the medium as necessary during the culture. When a transformant of the present invention comprises an inducible promoter, an inducer may be added into the medium as necessary. For instance, when *lac* promoter is contained, those such as isopropyl-β-D-thiogalactopyranoside, when *trp* promoter is contained, those such as an indoleacrylic acid may be added into the medium.
A transformant in culture is capable of expressing the subject heterologous gene with or without the induction, and accumulating the product in the culture. The expression product of a heterologous gene may be accumulated within the host cell, secreted outside the host cell, or accumulated on the outer membrane of the host cell, and the manner of the production may be altered by changing the host cell to be used or the structure of the expression product. In the present invention, a manner by which the expression product is secreted outside the host cell is preferred, because the purification of the expression product is easy.

The expression product produced by a transformant of the present invention may be purified as follows. For instance, in case when the expression product is expressed in the cell in dissolved form, after being cultured, the cells are collected by centrifugation and suspended in an aqueous buffer and fractured using a mechanical homogenization method by a French press, homogenizer or DYNO-MILL, freeze-thaw method, lysozyme addition method, ultrasonication method or surfactant addition method, either alone or in combination to give a cell-free extract, which subsequently is centrifuged to give a supernatant, from which the expression product may be purified using a ordinary method for isolating and purifying a protein, i.e., such as solvent extraction method, dialysis method, ultrafiltration method, gel filtration method, salt precipitation method with ammonium sulfate, etc., desalting method, precipitation method with an organic solvent, anion exchange chromatography method, cation exchange chromatography method, hydrophobic chromatography method, affinity chromatography method (e.g., metal chelate affinity chromatography method), chromatofocusing method, electrophoresis method (such as SDS-PAGE, agarose gel electrophoresis method, isoelectric point electrophoresis method), electroelution method, either alone or in combination.

In case when the expression product is accumulated within the cell as an insoluble form, the expression product may be purified as follows: the cells are collected, then fractured and centrifuged to collect the insoluble form of the polypeptide as a precipitated fraction, which is then solubilized with a protein denaturing agent, and the solution obtained is either diluted or dialyzed to bring the expression product back to its normal configuration before being subjected to isolation and purification methods as mentioned above.
In case when the expression product is secreted outside the cell, the expression product may be purified as follows: the culture is treated by centrifugation, etc. to give a soluble fraction, which is then subjected to isolation and purification methods as mentioned above.
In order to facilitate the collection of the expression product, a tag peptide having a binding ability to a particular substance may preliminarily be incorporated in the polypeptide to be expressed. Such tag peptide includes, such as, without limitation, His tag, GST tag, S tag and T7 tag. Thus, a subjected heterologous gene may comprise a nucleic acid sequence encoding for such a tag peptide.

The method of the present invention may be applied to various scale of production including from a small-scale production at a laboratory level to a large-scale production at a factory level. In each case, the aforementioned method of the present invention may appropriately be modified to be compatible to the scale. A modification compatible to each production scale is well known to those skilled in the art.

The present invention also relate to a kit for the production of a gene product, comprising a nucleic acid molecule, a nucleic acid construct, a vector and/or a host cell of the present invention.
The nucleic acid molecule, nucleic acid construct, vector and/or host cell of the present invention contained in the kit of the invention may be in any form as long as they exist in container(s) in a storable state, though, typically, they are stored in container(s) in form of a suspension in a storage solution or in a lyophilized form. When it is in a form of a suspension in a storage solution, a refrigerated or frozen storage may be necessary. The container preferably is sealed. Lyophilization may be carried out by any method known to those skilled in the art. Specifically, without limitation, for example, an object is suspended in a storage solution containing such as a dispersion medium, then the suspension is introduced into a sealable container (such as an ampule), which is then loaded into a lyophilizer for lyophilization. After finishing lyophilization, the container is sealed. Various operations such as production of the object, introduction into a container and lyophilization are carried out preferably under a sterile condition. Various storage solutions corresponding to the use and the object to be stored are known, and they may appropriately be used. Ingredients contained in a storage solution include, without limitation, e.g., propylene glycol, glycerol, polyethylene glycol, ethylene glycol, butanediol, formamide, propanediol, sorbitol, mannitol, DMSO, EDTA, Tris-HCl and TE (a mixture of Tris-HCl and EDTA).

A kit of the present invention may comprise one or more heterologous genes of interest as a nucleic acid construct operably linked to a nucleic acid molecule of the present invention or a vector comprising it, or alternatively may comprise a heterologous gene in another nucleic acid construct or vector other than the nucleic acid molecule of the present invention. Accordingly, the kit of the present invention may only comprise a nucleic acid construct or vector which comprises a nucleic acid molecule of the present invention but not a subject heterologous gene; or may only comprise a nucleic acid construct or vector which comprises a nucleic acid molecule of the present invention and a heterologous gene operably linked thereto; or may comprise a nucleic acid construct or vector which comprises a nucleic acid molecule of the present invention but not a subject heterologous gene and a nucleic acid construct or vector which comprises a subject heterologous gene but not a nucleic acid molecule of the present invention. A nucleic acid construct or vector which comprises a subject heterologous gene but not a nucleic acid molecule of the present invention may be provided separately as an optional item of the kit of the present invention. When a nucleic acid molecule of the present invention and a subject heterologous gene are comprised in separate nucleic acid constructs or vectors, each nucleic acid construct or vector may preferably be designed such that a nucleic acid construct or vector comprising a nucleic acid molecule of the present invention and a heterologous gene operably linked thereto is produced by treating these nucleic acid constructs or vectors with a restriction enzyme, etc.

A kit of the present invention may comprise elements useful for the production of a gene product other than those described above. Such elements include, without limitation, e.g., a reconstituent that reconstitutes the nucleic acid molecule, nucleic acid construct, vector and/or host cell of the present invention from the storage state, a restriction enzyme to be used for incorporation of the nucleic acid molecule, a culture medium for culturing the host cell, a selection medium for selecting the transformed host cell, a standard substance of the gene product of a known amount which will be a basis for assessing the expression level of the gene product, , an instruction or an electric recording medium such as CDs and DVDs describing a reconstitution method for nucleic acid molecule, nucleic acid construct, vector and/or host cell of the present invention, and/or a process for the production of a gene product.

### [EXAMPLES]

Hereinbelow, the present invention is further described based on specific examples, though such specific examples are merely exemplifications of the present invention and do not limit the present invention.

### Example 1: Cloning of C. perfringens τ-toxin a component (Ia) gene

### (1) Extraction of C. perfringens plasmid DNA

Type E *C. perfringens* (NCIB 10748) was cultured in 500 ml of the Brain Heart Infusion broth at 37°C for 6 hours, centrifuged at 8,000 rpm to collect the cells. Pelleted cell bodies were suspended in TNE buffer (100 mM NaCl, 100 mM EDTA, 10 mM Tris-HCI (pH 8)), washed by centrifugation. 25% sucrose-containing TNE buffer and lysozyme (10 mg/ml) was added to the pelleted cell bodies, which was shaken at 37°C for 15 minutes. Subsequently, EDTA was added to a final concentration of 100 mM, and the mixture was incubated at 37°C for 20 minutes. To this, N-Sodium lauroyl sarcosinate (LSS) was added to a final concentration of 1% and gently stirred, and then the lysate was left overnight at 4°C. After centrifuging at 15,000 rpm for 20 minutes, the supernatant was collected, which was made to 8 ml with TNE buffer and 8 g of cesium chloride was dissolved therein, centrifuged using a vertical rotor at 4°C, 45,000 rpm for 12 hours, and a fraction containing plasmid DNAs was isolated while confirming with an ultraviolet lamp. Obtained fraction was dialyzed with 3 1 of TE buffer, then plasmid DNAs were purified by ethanol precipitation method, dissolved in TE buffer (1 mM EDTA, 10 mM Tris-HCl (pH 8.0)) to give a plasmid DNA as a starting material for cloning.

### (2) Cloning of Ia gene

Using the DNA obtained as above as template, primers were generated referring to a known nucleotide sequence of Ia gene (Infect. Immun. 61, 5147-5156 (1993)), and a region comprising Ia structural gene and the upstream and downstream thereof was amplified by PCR. The primer sequences were as follows.
Forward primer: 5'-GAATTCAGAAAATACAATCT -3' (SEQ ID NO: 25)
Reverse primer: 5'-TATGATAACGTTTGACTTAT-3' (SEQ ID NO: 26)
Obtained PCR product of 1721 bp (SEQ ID NO: 27) was inserted into pT7BlueT-Vector (Novagen) using Taq DNA polymerase to give a vector having Ia gene, i.e., pT-Ia.

### (3) Determination of the nucleotide sequence of Ia gene

The obtained gene comprising the structural gene of Ia was about 1.3 kbp as confirmed by agarose gel electrophoresis. The whole sequence of pT-Ia was sequenced using Big dye reagent (Applied Biosystems) and the primers generated based on the Ia genetic sequence described in the reference above, and analyzed with ABI PRISM® 310 Genetic Analyzer (Applied Biosystems). The gene sequence of Ia was consistent with that described in the reference above.

### Example 2: Construction of a vector comprising Ia gene promoter region

From pT-Ia, a region containing the structural gene of Ia and the upstream and downstream thereof (SEQ ID NO: 27) was cut out with restriction enzymes EcoRI and XbaI, and inserted into a *E. coli-B. subtilis* shuttle vector pHY300PLK (TAKARA BIO INC.). The obtained vector pHY300PLK-CpIa, in which Ia gene has been inserted, contains a promoter region of Ia gene. In the downstream of this promoter region, i.e., between positions 1476 and 1477 of the complementary sequence of Ia gene sequence expressed by SEQ ID NO: 28, a multicloning sequence (SEQ ID NO: 29) was inserted. Specifically, an NdeI site (CATATG) was inserted into the above-mentioned site to generate a vector pHY300PLK-CpIa-NdeI, and then the above-mentioned multicloning site was cloned using Ligation kit (TAKARA BIO INC.). The obtained vector pHY300PLK-CpIa-MCS was subjected to the sequence analysis using ABI PRISM® 310 Genetic Analyzer (Applied Biosystems), confirming that a desired sequence (SEQ ID NO: 30) was obtained.

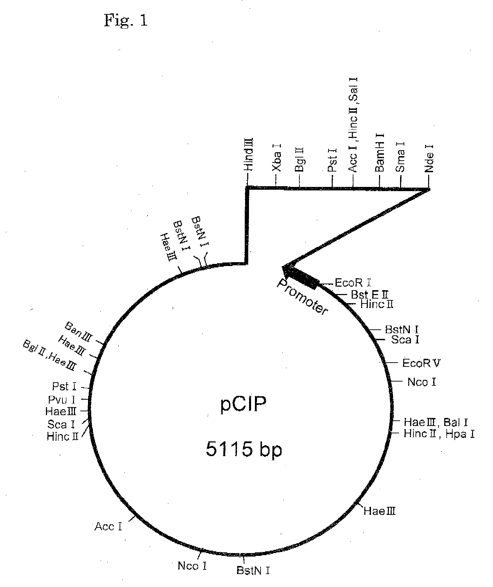

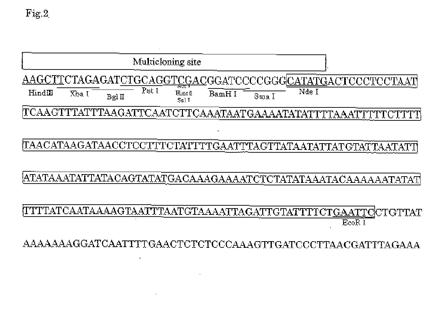

In another experiment, from pHY300PLK-CpIa-NdeI obtained as above, Ia gene promoter region (SEQ ID NO: 31) was cut out with NdeI and EcoRI, ligated into a modified pHY300PLK having a multicloning site with additional NdeI site on its 3' terminal (SEQ ID NO: 32), constructing a vector pCIP containing Ia gene promoter region. The obtained pCIP was subjected to the sequence analysis using ABI PRISM® 310 Genetic Analyzer (Applied Biosystems), confirming that a desired sequence expressed by SEQ ID NO: 33 was obtained (see, Figs. 1 and 2).

### Example 3: Cloning of heterologous genes

### (1) Cloning of C. perfringens α-toxin gene

### 1) Extraction of C. perfringens chromosomal DNA

Type A *C. perfringens* (NCTC8237) was cultured in 500 ml of the Brain Heart Infusion broth at 37°C for 6 hours, centrifuged at 8,000 rpm to collect the cells. The cell bodies were suspended in TNE buffer, and washed by centrifugation. The cell bodies were suspended in TNE buffer, which was shaken at 37°C for 60 minutes. To this, SDS was added to a final concentration of 1%, which was gently stirred, then an equal volume of saturated phenol-chloroform was added and the mixture was well mixed. After being centrifuged at 10,000 rpm for 10 minutes, the supernatant was collected. An equal volume of saturated chloroform-isoamyl alcohol was added to the supernatant and the mixture was well mixed, centrifuged at 10,000 rpm for 10 minutes, and the supernatant was collected and mixed with a double volume of 99% ethanol, and cooled at -30°C for 2 hours to precipitate DNAs. Subsequently, after being centrifuged at 10,000 rpm for 10 minutes, the supernatant was discarded, and the precipitation was dried under reduced pressure for 20 minutes. This precipitation was dissolved in TE buffer, added thereto an RNase (Sigma) solution to be 125 mg/ml, and treated at 37°C for 10 minutes. To this, NaCl was added to a final concentration of 0.1 M, and an equal volume of saturated chloroform-isoamyl alcohol was further added thereto, and the mixture was well stirred. After being centrifuged at 10,000 rpm for 10 minutes, 2.5 volumes of 99% ethanol was added to the supernatant, cooled at -80°C for 30 minutes. The precipitated DNA was centrifuged at 10,000 rpm for 10 minutes, and then the precipitation was dried under reduced pressure, dissolved in TE buffer to give a chromosomal DNA as a starting material for cloning.

### 2) Cloning of C. perfringens α-toxin gene

The chromosomal DNA above was cleaved with HindIII, a DNA fragment of 3 to 4 kbp was collected and ligated with pUC19 vector which had been cleaved with HindIII and dephosphorylated. This ligation solution was used for transforming *E. coli* JM109, which was inoculated onto a yolk agar medium and cultured overnight. Colonies showing white turbidity in their periphery due to the expression of α-toxin gene were picked up, inoculated and cultured in an LB medium, then a plasmid contained α-toxin gene (pUA-3.1) was isolated.

### 3) Determination of the nucleotide sequence of C. perfringens α-toxin gene

The obtained gene containing the structural gene of α-toxin was about 3.1 kbp, as confirmed by agarose gel electrophoresis. The whole sequence of pUA-3.1 was sequenced using Big dye reagent (Applied Biosystems) and primers generated based on previously, reported gene sequence of α-toxin (Infect. Immun. 57, 367-376 (1989)), analyzed using ABI PRISM® 310 Genetic Analyzer (Applied Biosystems). The obtained gene sequence of α-toxin was consistent with that described in the reference above.

### 4) Cloning of C. perfringens α-toxin gene into an expression vector

Using pUA-3.1 as template, a region of 1.3 kbp comprising the structural gene of α-toxin and the upstream and downstream thereof was amplified by PCR. The primer sequences were as follows.
Forward primer: -5'-TTTAAAAAATATTCAAAAAAT-3' (SEQ ID NO: 34)
Reverse primer: 5'-GGAAGCTTTTATTTTGTAAATAC-3' (SEQ ID NO: 35)
The obtained PCR product of 1.3 kbp (SEQ ID NO: 36) was made blunt-ended, inserted into pHY300PLK which had been cleaved with SmaI, to give *C. perfringens* α-toxin gene expression vector pHY300PLK-Cpα.

### (2) Cloning of C. perfringens β2-toxin gene

### 1) Extraction of C. perfringens plasmid DNA

Type A *C. perfringens* (Strain 13) was cultured in 500 ml of the Brain Heart Infusion broth at 37°C for 6 hours, centrifuged at 8,000 rpm to collect the cells. Pelleted cell bodies were suspended in TNE buffer (100 mM NaCI, 100 mM EDTA, 10 mM Tris-HCl (pH 8)), and washed by centrifugation. 25% sucrose-containing TNE buffer and lysozyme (10 mg/ml) was added to the pelleted cell bodies, which was shaken at 37°C for 15 minutes. Subsequently, EDTA was added to a final concentration of 100 mM, and the mixture was incubated at 37°C for 20 minutes. To this, N-Sodium lauroyl sarcosinate (LSS) was added to a final concentration of 1% and gently stirred, and then the lysate was left overnight at 4°C. After centrifuging at 15,000 rpm for 20 minutes, the supernatant was collected, which was made to 8 ml with TNE buffer and 8 g of cesium chloride was dissolved therein, centrifuged using a vertical rotor at 4°C, 45,000 rpm for 12 hours, and a fraction containing plasmid DNAs was isolated while confirming with an ultraviolet lamp. Obtained fraction was dialyzed with 3 1 of TE buffer, then plasmid DNAs were purified by ethanol precipitation method, dissolved in TE buffer (1 mM EDTA, 10 mM Tris-HCl (pH 8.0)) to give a plasmid DNA as a starting material for cloning.

### 2) Cloning of C. perfringens β2-toxin gene

Using the DNA obtained as above as template, primers were generated referring to a known nucleotide sequence of β2-toxin gene (Gene 203, 65-73 (1997)), (forward primer: 5'-TTAGATAAAAGTGTAAAAGA-3' (SEQ ID NO: 37), reverse primer: 5'-TTAGGTTTTTATATAATAA-3' (SEQ ID NO: 38)), and a region comprising the structural gene of β2-toxin and the upstream and downstream thereof was amplified by PCR. A PCR product of 960 bp (SEQ ID NO: 39) was inserted into pT7Blue vector to give a vector having β2-toxin gene, i.e., pT-β2.

### (5) Cloning of Bacillus cereus sphingomyelinase (BcSMase) gene

### 1) Extraction of B. cereus plasmid DNA

*B. cereus* (IAM1029) was cultured in 500 ml of the Brain Heart Infusion broth at 37°C for 6 hours, centrifuged at 8,000 rpm to collect the cells. Pelleted cell bodies were suspended in TNE buffer (100 mM NaCl, 100 mM EDTA, 10 mM Tris-HCl (pH 8)), and washed by centrifugation. 25% sucrose-containing TNE buffer and lysozyme (10 mg/ml) was added to the pelleted cell bodies, which was shaken at 37°C for 15 minutes. Subsequently, EDTA was added to a final concentration of 100 mM, and the mixture was incubated at 37°C for 20 minutes. To this, N-Sodium lauroyl sarcosinate (LSS) was added to a final concentration of 1% and gently stirred, then the lysate was left overnight at 4°C. After centrifuging at 15,000 rpm for 20 minutes, the supernatant was collected, which was made to 8 ml with TNE buffer and 8g of cesium chloride was dissolved therein, centrifuged using a vertical rotor at 4°C, 45,000 rpm for 12 hours, a fraction containing plasmid DNAs was isolated while confirming with an ultraviolet lamp. Obtained fraction was dialyzed with 3 1 of TE buffer, then plasmid DNAs were purified by ethanol precipitation method, dissolved in TE buffer (1 mM EDTA, 10 mM Tris-HCl (pH 8.0)) to give a plasmid DNA as a starting material for cloning.

### 2) Cloning of B. cereus sphingomyelinase gene

Using the DNA obtained as above as template, *B. cereus* sphingomyelinase gene was amplified by PCR using a forward primer: 5'-ATGGAGGTATGGAACGTG-3' (SEQ ID NO: 40) and a reverse primer: 5'-CTACTTCATAGAAATAGT-3' (SEQ ID NO: 41). A PCR product (SEQ ID NO: 42) was inserted into pT7Blue vector to give a vector having *B. cereus* sphingomyelinase gene, i.e., pT-BcSMase.

### Example 4: Insertion of a heterologous gene into pCIP vector

Into the multicloning site of pCIP generated in Example 2, a heterologous gene obtained in Example 3 (*C. perfringens* α-toxin gene, *C. perfringens* β2-toxin gene or *B. cereus* sphingomyelinase gene), *C. botulinum* α-toxin (phospholipase C (CbPLC)) gene or *C. botulinum* C3 enzyme gene was inserted.

### (1) Insertion of C. perfringens α-toxin gene into pCIP

Using pUA-3.1 as template, a region of 1.3 kbp containing the structural gene of α-toxin and the upstream and downstream thereof was amplified by PCR. The primer sequences were as follows.
Forward primer: 5'-GGGCATATGATGAAAAGAAAGATTTGTAAG-3' (SEQ ID NO: 43)
Reverse primer: 5'-CCCTCTAGATTATTTTATATTATAAGTTGA-3' (SEQ ID NO: 44)
The obtained gene of about 1.2 kbp (SEQ ID NO: 45) was cleaved with NdeI and XbaI, ligated with pCIP that had been cleaved with the same enzymes, to give *C. perfringens* α-toxin gene expression vector pCIP-Cpα.

### (2) Insertion of C. perfringens β2-toxin gene into pCIP

Using pT-β2 as template, a region of about 0.8 kbp containing the structural gene of β2-toxin and the upstream and downstream thereof was amplified by PCR. As primers, a forward primer having a leading NdeI restriction site and thereafter a sequence following the start codon: 5'-AGGCATATGAAAAAAATTATTTCAAAGTTT-3' (SEQ ID NO: 46), and a reverse primer comprising the downstream 29 bps that terminates at a stop codon and having XbaI site at its end: 5'-CCTCTAGACTATGCACAATATCCTTC-3' (SEQ ID NO: 47) were used. The obtained gene of about 0.8 kbp (SEQ ID NO: 48) was cleaved with NdeI and XbaI, ligated with pCIP that had been cleaved with the same enzymes, to give pCIP-Cpβ2.

### (3) Insertion of B. cereus sphingomyelinase gene into pCIP

Using pT-BcSMase as template, a forward primer having a leading NdeI restriction site: 5'-CATATGATGGAGGTATGGAACGTG-3' (SEQ ID NO: 49), and a reverse primer having an XbaI site at its end: 5'TCTAGACTACTTCATAGAAATAGT-3' (SEQ ID NO: 50) were used to amplify sphingomyelinase gene by PCR. The obtained gene of about 1.0 kb (SEQ ID NO: 51) was cleaved with NdeI and XbaI, ligated with pCIP that had been cleaved with the same enzyme to give pCIP-BcSMase.

### (4) Insertion of C. botulinum phospholipase C (CbPLC) gene into pCIP

Using *C. botulinum* phospholipase C gene inserted into pUC18 vector (pUC18-BPLC, provided from Prof. Keiji Oguma of Okayama University, Graduate School) as template, a region of about 1.2 kbp containing the structural gene of CbPLC enzyme and the upstream and downstream thereof was amplified by PCR. As primers, a forward primer starting with a start codon and having a leading NdeI restriction site: 5'-CATATGATGAATAAGAAAAAAATATTAAAA-3' (SEQ ID NO: 52), and a reverse primer having a stop codon and an XbaI site downstream thereto: 5'-TCTAGATTATTTATTATTTATATAGAATGT-3' (SEQ ID NO: 53) were used. The obtained gene of about 1.2 kb (SEQ ID NO: 54) was cleaved with NdeI and XbaI, ligated with pCIP that had been cleaved with the same enzymes to give pCIP-CbPLC.

### (5) Insertion of C. botulinum C3 enzyme gene into pCIP

Using C3 enzyme gene inserted into pUC18 vector (pUC18-C3, provided from Prof. Keiji Oguma of Okayama University, Graduate School) as template, a region of about 0.8 kbp containing the structural gene of C3 enzyme and the upstream and downstream thereof was amplified by PCR. As primers, a forward primer starting with a start codon and having a leading NdeI restriction site: 5'-GGCATATGAAAGGTTTAAGAAAATCA-3' (SEQ ID NO: 55), and a reverse primer having a stop codon and an XbaI site downstream thereto: 5'-CCTCTAGATTATTTAGGATTGATAGCTGT-3' (SEQ ID NO: 56) were used. The obtained gene of about 0.8 kb (SEQ ID NO: 57) was cleaved with NdeI and XbaI, ligated with pCIP that had been cleaved with the same enzymes to give pCIP-CbC3.
Each of the obtained vectors was sequenced with ABI PRISM® 310 Genetic Analyzer (Applied Biosystems), confirming that the desired sequences expressed by SEQ ID NOs: 58 to 62 were obtained.

### Example 5: A heterologous gene expression

### (1) Culturing of the host B. subtilis cell

*B. subtilis* ISW1214 strain was pre-cultured overnight in 2 ml LB (Luria-Broth) medium at 37°C. 2 ml of the pre-culture was added to 32 ml of the main-culture (32 ml sterile LB medium supplemented with 2.9 g sorbitol and sterilized by an autoclave), and main culture was performed at 37°C (about 3 hours). When the optical density (A₆₂₀) reached from 0.85 to 0.95, the main culturing was ended, the culture was left in ice for 10 minutes and then centrifuged at 5,000 x g (9500 rpm) for 5 minutes. Subsequently, the cell bodies were washed 4 times with an ice-cooled Solution A (0.5 M sorbitol +0.5 M mannitol +10% glycerol: 45.5 g sorbitol, 45.5 g mannitol and 50 ml glycerol were mixed, messed up with distilled water to 500 ml, then sterilized), then suspended in 0.8 ml (i.e., 1/40 volume of the culture solution) of Solution A. The obtained bacterial suspension was dispensed into sterile tubes (60 µl each) and stored at -80°C.

### (2) Introduction of a vector

Each of the vectors generated in Examples 2 to 4 (pHY300PLK-CpIa, pHY300PLK-Cpα, pCIP-Cpα, pCIP-Cpβ2, pCIP-CbPLC, pCIP-CbC3 or pCIP-BcSMase) was introduced into a host *B. subtilis* cell by electroporation method. The electroporation was performed as follows. First, 60 µl of the bacterial suspension for electroporation prepared in (1) above was thawed in ice, and 1µl vector solution (100 ng/µl) was added thereto. This mixture was transferred to an ice-cooled 0.1 cm cuvette and left for 1 to 1.5 minutes before pulsing (2 kV, 200 Ω, 25 mF) by a gene-introducing device (BTX, ECM-630). Subsequently, this was transferred to a 15 ml centrifugation tube, 1 ml of Solution B (LB medium +0.5 M sorbitol +0.38 M mannitol) was added thereto, and cultured at 37°C for 3 hours. The culture was centrifuged (3500 rpm, 5 minutes), 900 µl of the supernatant was removed, the pellet was suspended and plated onto a LB agar medium containing tetracycline (30 mg/ml), and cultured overnight at 37°C. Formed resistant colonies of the transformants were picked up, and subjected to the culturing as follows.

### (3) Culturing of the transformant and collection/purification of the gene product

The transformant transfected with either of the vectors was cultured while stirring in 11 LB medium at 37°C for 14 hours, then centrifuged at 4°C, 8,000 rpm for 20 minutes. To the culture supernatant, ammonium sulfate (Nacalai Tesque, 02619-86) was added at regular intervals in small amount while ice cooling and stirring to make a saturated ammonium sulfate (472 g/l) of a final concentration of 70%, which was left overnight. It was then centrifuged at 4°C, 9,500 rpm for 30 minutes, the resulting pellet was dissolved in 0.02 M TB buffer (0.02 M Tris/HCl, pH 7.5), dialyzed overnight with the same buffer at 4°C. After dialysis, it was centrifuged at 4°C, 15,000 rpm for 30 minutes to give a supernatant as a crude product (ammonium sulfate product) sample. This crude product sample was diluted with 1 M NaCl-TB (pH 7.5) to make the final concentration of NaCl to be 0.5M, then purified using copper-chelating affinity column (Chelating Sepharose Fast Flow, GE healthcare), anion exchange column (UNO™ Q-1 R Column, BIO-RAD, 720-0011) and/or cation exchange column (SP-TOYOPEARL 650 M, Tosoh Corporation). Each of the obtained products was measured for the protein content by BCA method and confirmed for the absence of impurity by SDS-PAGE. A comparison of the final amount purified from 1 1 of culture and the maximum final amount purified from 1 1 of culture using similar procedures using conventional pHY300PLK is shown below.

**[Table 1]**

| Vector name | Purification column | Yield (mg) | Conventional yield (mg) |
|---|---|---|---|
| pHY300PLK - CpIa | P | 42.8 | - |
| pCIP - Cpα | Cu→N | 48.5 | 12 |
| pCIP - Cpβ2 | Cu→N | 25.3 | 8 |
| pCIP - CbPLC | Cu→N | 23.7 | 3 |
| pCIP - CbC3 | P | 45.6 | 5 |
| pCIP - BcSMase | Cu→N | 58.6 | 5 |

| | | | |
|---|---|---|---|
| Purification column: P (cation exchange column), N (anion exchange column), Cu (copper-chelating affinity column) | | | |

The results described above indicate that a vector of the present invention comprising a promoter region of *C. perfringens τ*-toxin gene significantly increases the heterologous gene expression. Also, since *C. perfringens* α and β2-toxin gene were highly expressed in *B. subtilis* cell using a conventional vector, it was suggested that the promoter of said gene may increase the heterologous gene expression.

## Claims

1. A nucleic acid molecule comprising a promoter region derived from a *Clostridium* toxin gene, which enhances the expression of a heterologous gene operably linked thereto.

2. The nucleic acid molecule according to Claim 1, wherein the toxin gene is selected from the group consisting of *C. perfringens* α-toxin, ε-toxin and τ-toxin, botulinum toxin, and tetanus toxin.

3. A nucleic acid construct comprising the nucleic acid molecule according to Claim 1 or 2 and a heterologous gene operably linked thereto.

4. A vector comprising the nucleic acid molecule according to Claim 1 or 2 or the nucleic acid construct according to Claim 3.

5. A host cell transformed with the vector according to Claim 4.

6. A process for the production of a gene product, comprising a step of culturing the host cell according to Claim 5.

7. A kit for the production of a gene product, comprising the nucleic acid molecule according to Claim 1 or 2, the nucleic acid construct according to Claim 3, the vector according to Claim 4 and/or the host cell according to Claim 5.


SEQUENCE LISTING

<110>  Otsuka Chemical Co., Ltd.

<120>  High-expression promoter and method for producing gene product
       using the same

<130>  PCT2353OC

<150>  JP2009-007950
<151>  2009-1-16

<160>  62

<170>  PatentIn version 3.5

<210>  1
<211>  784
<212>  DNA
<213>  Clostridium perfringens

<400>  1
gaattccaag accatcgata ccttcactta ttatatattc tctaacgata ggtgcaaggg      60

cagcctttcc atctccacct cttgaataga ttgttctacc agaccctttt aattaacatc     120

atatctttta ctatctttag ttacatgttc tcctaataag agtgctctac catctcctag     180

cattgtaaaa tgaccaaatt ggtcgcccag cataagcctg tgcaattggt acaatcctgg     240

aagttcattc ctgcaaatat attaagtcca aaatcgctgt ttaaaacttc ttcattaagc     300

ccaagttctt cagcaagaga agtattaaac ttaataagtt taggattttt tgaacccttt     360

ggattttgtt cactaaagaa tatatttgga agagttaaat aagtgttttc taagttaaaa     420

cctgtttttg attgaaaatt tttattatcc atattaaaat cctttgcctt ataatttatt     480

tcaaatttta tccatccctt atattatgtg taaaaattct tattaaatta aaaaacaaga     540

tttaacttat tatagcacta ataattgtaa attttcatat taaaaataag tttaacaatt     600

tagagtgggt aaggttagat atgtttaatt gaaatttgaa ttgtattcaa aaatatttta     660

aaaaatattc aaaaatttag tgagcttatg gtaattatat ggtataattt cagtggcaag     720

tgttaatcgg ttatcaaaaa aggggagatt aatacttgaa aaaaattaac gggggatata     780

aaaa                                                                  784


<210>  2
<211>  187
<212>  DNA
<213>  Clostridium perfringens

<400>  2
gatcgttttt agttctattt aaataaacga tttaataata aaaattttta acttgggttt      60

tgtcgtaaat gttggagcta ccccaatata ataaaatttg tatattaaat aattttattt     120

atattattta ctttttttaa aaaatataga aaaatataga aaaatatatt aatgaaaggg 180

tggtttt 187

<210> 3
<211> 245
<212> DNA
<213> Clostridium perfringens

<400> 3
gaattcagaa aatacaatct aattttacat taaattactt ttattgataa aaatatattt 60

tttgtattta tatagagatt ttctttgtca tatactgtat aatatttata taatattaat 120

acataatatt ataactaaat tcaaaataga aaggaggtta tcttatgtta aaaagaaaaa 180

tttaaaatat attttcatta tttgaagatt gaatcttaaa taaacttgaa ttaggaggga 240

gtatt 245

<210> 4
<211> 76
<212> DNA
<213> Clostridium botulinum

<400> 4
tcaaagtatt tgtatttatg gtcatttaaa taattaataa tttaattaat tttaaatatt 60

ataagaggtg ttaaat 76

<210> 5
<211> 227
<212> DNA
<213> Clostridium botulinum

<400> 5
gaattcaact agtagataac aaaaataatg cacagatttt tattattaat aatgatatat 60

ttatctctaa ctgtttaact ttaacttata acaatgtaaa tgtatatttg tctataaaaa 120

atcaagatta caattgggtt atatgtgatc ttaatcatga tataccaaaa aagtcatatc 180

tatggatatt aaaaaatata taaatttaaa attaggagat gctgtat 227

<210> 6
<211> 280
<212> DNA
<213> Clostridium tetani

<400> 6
tagcattaaa aaaattagaa cctatagtaa ataaattaat taatatatag tttttataat 60

ttaattatga ataatattct taagataaaa agtaaatttt taaaaattta aattttcagt 120

ttacaaaaaa taacctgatt atgttatatg taattgtaaa aaacatataa aaaatcagaa 180

aaatttagga ggtatattat taatggatta aataataatt ttttaattta cttttgatta 240

ataaatatta aatgtttatt ttaattagga gatgatacgt 280

<210> 7
<211> 29
<212> DNA
<213> Clostridium perfringens

<400> 7
tgagcttatg gtaattatat ggtataatt 29

<210> 8
<211> 29
<212> DNA
<213> Clostridium perfringens

<400> 8
tttgtatatt aaataatttt atttatatt 29

<210> 9
<211> 30
<212> DNA
<213> Clostridium perfringens

<400> 9
ttgtcatata ctgtataata tttatataat 30

<210> 10
<211> 28
<212> DNA
<213> Clostridium botulinum

<400> 10
tggtcattta aataattaat aatttaat 28

<210> 11
<211> 6
<212> DNA
<213> Clostridium botulinum

<400> 11
tatatt 6

<210> 12
<211> 8
<212> DNA
<213> Clostridium tetani

<400> 12
tatattat 8

<210>  13
<211>  104
<212>  DNA
<213>  Clostridium perfringens

<400>  13
tgagcttatg gtaattatat ggtataattt cagtggcaag tgttaatcgg ttatcaaaaa      60

aggggagatt aatacttgaa aaaaattaac gggggatata aaaa                      104


<210>  14
<211>  91
<212>  DNA
<213>  Clostridium perfringens

<400>  14
tttgtatatt aaataatttt atttatatta tttacttttt ttaaaaaata tagaaaaata      60

tagaaaaata tattaatgaa agggtggttt t                                     91


<210>  15
<211>  161
<212>  DNA
<213>  Clostridium perfringens

<400>  15
ttgtcatata ctgtataata tttatataat attaatacat aatattataa ctaaattcaa      60

aatagaaagg aggttatctt atgttaaaaa gaaaaattta aaatatattt tcattatttg     120

aagattgaat cttaaataaa cttgaattag gagggagtat t                         161


<210>  16
<211>  58
<212>  DNA
<213>  Clostridium botulinum

<400>  16
tggtcattta aataattaat aatttaatta attttaaata ttataagagg tgttaaat        58


<210>  17
<211>  125
<212>  DNA
<213>  Clostridium botulinum

<400>  17
tatatttgtc tataaaaaat caagattaca attgggttat atgtgatctt aatcatgata      60

taccaaaaaa gtcatatcta tggatattaa aaaatatata aatttaaaat taggagatgc     120

tgtat                                                                 125


<210>  18
<211>  88
<212>  DNA

<213>  Clostridium tetani

<400>  18
tatattatta atggattaaa taataatttt ttaatttact tttgattaat aaatattaaa      60

tgtttatttt aattaggaga tgatacgt                                          88


<210>  19
<211>  95
<212>  DNA
<213>  Clostridium perfringens

<400>  19
tgagcttatg gtaattatat ggtataattt cagtggcaag tgttaatcgg ttatcaaaaa      60

aggggagatt aatacttgaa aaaaattaac ggggg                                  95


<210>  20
<211>  87
<212>  DNA
<213>  Clostridium perfringens

<400>  20
tttgtatatt aaataatttt atttatatta tttacttttt ttaaaaaata tagaaaaata      60

tagaaaaata tattaatgaa agggtgg                                           87


<210>  21
<211>  154
<212>  DNA
<213>  Clostridium perfringens

<400>  21
ttgtcatata ctgtataata tttatataat attaatacat aatattataa ctaaattcaa      60

aatagaaagg aggttatctt atgttaaaaa gaaaaattta aaatatattt tcattatttg     120

aagattgaat cttaaataaa cttgaattag gagg                                  154


<210>  22
<211>  50
<212>  DNA
<213>  Clostridium botulinum

<400>  22
tggtcattta aataattaat aatttaatta attttaaata ttataagagg                  50


<210>  23
<211>  117
<212>  DNA
<213>  Clostridium botulinum

<400>  23
tatatttgtc tataaaaaat caagattaca attgggttat atgtgatctt aatcatgata      60

taccaaaaaa gtcatatcta tggatattaa aaaatatata aatttaaaat taggaga 117

<210> 24
<211> 80
<212> DNA
<213> Clostridium tetani

<400> 24
tatattatta atggattaaa taataatttt ttaatttact tttgattaat aaatattaaa 60

tgtttatttt aattaggaga 80

<210> 25
<211> 20
<212> DNA
<213> Artificial sequence

<220>
<223> CpIa forward primer

<400> 25
gaattcagaa aatacaatct 20

<210> 26
<211> 20
<212> DNA
<213> Artificial sequence

<220>
<223> CpIa reverse primer

<400> 26
tatgataacg tttgacttat 20

<210> 27
<211> 1721
<212> DNA
<213> Clostridium perfringens

<400> 27
gaattcagaa aatacaatct aattttacat taaattactt ttattgataa aaatatattt 60

tttgtattta tatagagatt ttctttgtca tatactgtat aatatttata taatattaat 120

acataatatt ataactaaat tcaaaataga aaggaggtta tcttatgtta aaaagaaaaa 180

tttaaaatat attttcatta tttgaagatt gaatcttaaa taaacttgaa ttaggaggga 240

gtattatgaa aaaagttaat aaatctatat ctgtatttct aatattatat ttaattttaa 300

ctagttcatt tcctagttat acttatgcac aagatttaca aatagcaagc aattatatta 360

cagatagagc ttttattgaa agaccagaag attttcttaa agataaagaa aatgctattc 420

aatgggaaaa aaaggaggct gaaagagtag aaaaaaacct tgatacactt gaaaaagaag 480

cattagaatt atataaaaaa gattctgaac aaataagtaa ctactctcag acaagacagt 540

atttttacga ctatcaaata gaatcaaatc ctagagaaaa agaatacaaa aatcttagaa 600

atgccatatc aaaaaataag atagataaac ctataaatgt ttattatttt gagtctccag 660

agaaatttgc gtttaataaa gaaataagaa cagaaaatca aaatgaaatt tctttagaga 720

aatttaatga gttgaaagaa actattcaag ataaattgtt taaacaagat ggatttaagg 780

atgtttcttt atatgaacca ggtaatggcg atgaaaagcc tacaccacta cttatacatt 840

tgaaattacc aaaaaatact ggtatgttac catatataaa ttctaatgat gtaaaaacat 900

taatagaaca agactatagc ataaagatag acaaaattgt tcgtatagta atagaaggaa 960

agcaatatat aaaagctgaa gcttctattg taaacagtct tgattttaaa gatgatgtaa 1020

gtaaaggtga tttatgggga aaagaaaatt atagtgattg gagtaataaa ttaactccta 1080

atgaacttgc tgatgtaaat gactatatgc gtggaggata taccgcaatt aataactatt 1140

taatatcaaa tggtccttta aataatccta atccagaact agactctaaa gtaaataaca 1200

ttgaaaacgc attaaagctc acacctattc catctaactt aattgtatat agaaggtctg 1260

gtccacaaga atttggatta actctcacat ctcctgaata tgattttaat aaaatagaaa 1320

atatagatgc ttttaaagaa aaatgggaag gaaaagtaat aacataccca aactttatta 1380

gtactagtat tggaagtgta aatatgagtg catttgctaa aagaaaaata atactacgta 1440

taaacatacc aaaagattct ccaggagctt atttatcagc cattccaggt tatgcaggag 1500

aatatgaagt acttttaaat catggaagta aatttaaaat caataaagtt gattcttata 1560

aagatggaac tgtaacaaaa ctaattttgg atgcaacatt gataaattaa tacttttaat 1620

ataaataatc aatttaaagg aggaaaaata atgaatatac aaattaaaaa tgtatttagt 1680

tttttaacac ttacagctat gataagtcaa acgttatcat a 1721

<210> 28
<211> 1721
<212> DNA
<213> Clostridium perfringens

<400> 28
tatgataacg tttgacttat catagctgta agtgttaaaa aactaaatac atttttaatt 60

tgtatattca ttatttttcc tcctttaaat tgattattta tattaaaagt attaatttat 120

caatgttgca tccaaaatta gttttgttac agttccatct ttataagaat caactttatt 180

gattttaaat ttacttccat gatttaaaag tacttcatat tctcctgcat aacctggaat 240

ggctgataaa taagctcctg gagaatcttt tggtatgttt atacgtagta ttatttttct 300

tttagcaaat gcactcatat ttacacttcc aatactagta ctaataaagt ttgggtatgt 360

tattactttt ccttcccatt tttctttaaa agcatctata ttttctattt tattaaaatc 420

atattcagga gatgtgagag ttaatccaaa ttcttgtgga ccagaccttc tatatacaat 480

taagttagat ggaataggtg tgagctttaa tgcgttttca atgttattta ctttagagtc 540

tagttctgga ttaggattat ttaaaggacc atttgatatt aaatagttat taattgcggt 600

atatcctcca cgcatatagt catttacatc agcaagttca ttaggagtta atttattact 660

ccaatcacta taattttctt ttccccataa atcaccttta cttacatcat ctttaaaatc 720

aagactgttt acaatagaag cttcagcttt tatatattgc tttccttcta ttactatacg 780

aacaattttg tctatcttta tgctatagtc ttgttctatt aatgttttta catcattaga 840

atttatatat ggtaacatac cagtattttt tggtaatttc aaatgtataa gtagtggtgt 900

aggcttttca tcgccattac ctggttcata taaagaaaca tccttaaatc catcttgttt 960

aaacaattta tcttgaatag tttctttcaa ctcattaaat ttctctaaag aaatttcatt 1020

ttgattttct gttcttattt ctttattaaa cgcaaatttc tctggagact caaaataata 1080

aacatttata ggtttatcta tcttattttt tgatatggca tttctaagat ttttgtattc 1140

tttttctcta ggatttgatt ctatttgata gtcgtaaaaa tactgtcttg tctgagagta 1200

gttacttatt tgttcagaat cttttttata taattctaat gcttcttttt caagtgtatc 1260

aaggtttttt tctactcttt cagcctcctt tttttcccat tgaatagcat tttctttatc 1320

tttaagaaaa tcttctggtc tttcaataaa agctctatct gtaatataat tgcttgctat 1380

ttgtaaatct tgtgcataag tataactagg aaatgaacta gttaaaatta aatataatat 1440

tagaaataca gatatagatt tattaacttt tttcataata ctccctccta attcaagttt 1500

atttaagatt caatcttcaa ataatgaaaa tatattttaa atttttcttt ttaacataag 1560

ataacctcct ttctattttg aatttagtta taatattatg tattaatatt atataaatat 1620

tatacagtat atgacaaaga aaatctctat ataaatacaa aaaatatatt tttatcaata 1680

aaagtaattt aatgtaaaat tagattgtat tttctgaatt c 1721

<210> 29
<211> 42
<212> DNA
<213> Artificial sequence

<220>
<223> MCS for pHY300PLK-Ia-MCS

<400> 29
catatgcccg ggggatccgt cgacctgcag agatcttcta ga 42

<210>  30
<211>  6626
<212>  DNA
<213>  Artificial sequence

<220>
<223>  pHY300PLK-Ia-MCS

<400>  30
aagcttctag aggatctact agtcatatgg atttatgata acgtttgact tatcatagct        60

gtaagtgtta aaaaactaaa tacattttta atttgtatat tcattatttt tcctccttta       120

aattgattat ttatattaaa agtattaatt tatcaatgtt gcatccaaaa ttagttttgt       180

tacagttcca tctttataag aatcaacttt attgatttta aatttacttc catgatttaa       240

aagtacttca tattctcctg cataacctgg aatggctgat aaataagctc ctggagaatc       300

ttttggtatg tttatacgta gtattatttt tcttttagca aatgcactca tatttacact       360

tccaatacta gtactaataa agtttgggta tgttattact tttccttccc attttctttt       420

aaaagcatct atatttctta ttttattaaa atcatattca ggagatgtga gagttaatcc       480

aaattcttgt ggaccagacc ttctatatac aattaagtta gatggaatag gtgtgagctt       540

taatgcgttt tcaatgttat ttactttaga gtctagttct ggattaggat tatttaaagg       600

accatttgat attaaatagt tattaattgc ggtatatcct ccacgcatat agtcatttac       660

atcagcaagt tcattaggag ttaatttatt actccaatca ctataatttt cttttcccca       720

taaatcacct ttacttacat catctttaaa atcaagactg tttacaatag aagcttcagc       780

ttttatatat tgctttcctt ctattactat acgaacaatt ttgtctatct ttatgctata       840

gtcttgttct attaatgttt ttacatcatt agaatttata tatggtaaca taccagtatt       900

ttttggtaat ttcaaatgta taagtagtgg tgtaggcttt tcatcgccat tacctggttc       960

atataaagaa acatccttaa atccatcttg tttaaacaat ttatcttgaa tagtttcttt      1020

caactcatta aatttctcta aagaaatttc attttgattt tctgttctta tttctttatt      1080

aaacgcaaat ttctctggag actcaaaata ataaacattt ataggtttat ctatcttatt      1140

ttttgatatg gcatttctaa gatttttgta ttctttttct ctaggatttg attctatttg      1200

atagtcgtaa aaatactgtc ttgtctgaga gtagttactt atttgttcag aatctttttt      1260

atataattct aatgcttctt tttcaagtgt atcaaggttt ttttctactc tttcagcctc      1320

ctttttttcc cattgaatag cattttcttt atctttaaga aaatcttctg gtctttcaat      1380

aaaagctcta tctgtaatat aattgcttgc tatttgtaaa tcttgtgcat aagtataact      1440

aggaaatgaa ctagttaaaa ttaaatataa tattagaaat acagatatag atttattaac      1500

ttttttcatc atatgcccgg gggatccgtc gacctgcaga gatcttctag aaatactccc      1560

tcctaattca agtttattta agattcaatc ttcaaataat gaaaatatat tttaaatttt 1620

tctttttaac ataagataac ctcctttcta ttttgaattt agttataata ttatgtatta 1680

atattatata aatattatac agtatatgac aaagaaaatc tctatataaa tacaaaaaat 1740

atatttttat caataaaagt aatttaatgt aaaattagat tgtattttct gaattcctgt 1800

tataaaaaaa ggatcaattt tgaactctct cccaaagttg atcccttaac gatttagaaa 1860

tccctttgag aatgtttata tacattcaag gtaaccagcc aactaatgac aatgattcct 1920

gaaaaaagta ataacaaatt actatacaga taagttgact gatcaacttc cataggtaac 1980

aacctttgat caagtaaggg tatggataat aaaccaccta caattgcaat acctgttccc 2040

tctgataaaa agctggtaaa gttaagcaaa ctcattccag caccagcttc ctgctgtttc 2100

aagctacttg aaacaattgt tgatataact gttttggtga acgaaagccc acctaaaaca 2160

aatacgatta taattgtcat gaaccatgat gttgtttcta aaagaaagga agcagttaaa 2220

aagctaacag aaagaaatgt aactccgatg tttaacacgt ataaaggacc tcttctatca 2280

acaagtatcc caccaatgta gccgaaaata atgacactca ttgttccagg gaaaataatt 2340

acacttccga tttcggcagt acttagctgg tgaacatctt tcatcatata aggaaccata 2400

gagacaaacc ctgctactgt tccaaatata attcccccac aaagaactcc aatcataaaa 2460

ggtatatttt tccctaatcc gggatcaaca aaaggatctg ttactttcct gatatgtttt 2520

acaaatatca ggaatgacag cacgctaacg ataagaaaag aaatgctata tgatgttgta 2580

aacaacataa aaaatacaat gcctacagac attagtataa ttcctttgat atcaaaatga 2640

ccttttatcc ttacttcttt ctttaataat ttcataagaa acggaacagt gataattgtt 2700

atcataggaa tgagtagaag ataggaccaa tgaatataat gggctatcat tccaccaatc 2760

gctggaccga ctccttctcc catggctact atcgatccaa taagaccaaa tgctttaccc 2820

ctattttcct ttggaatata gcgcgcaact acaaccatta cgagtgctgg aaatgcagct 2880

gcaccagccc cttgaataaa acgagccata ataagtaagg aaaagaaaga atggccaaca 2940

aacccaatta ccgacccgaa acaatttatt ataattccaa ataggagtaa ccttttgatg 3000

cctaattgat cagatagctt tccatataca gctgttccaa tggaaaaggt taacataaag 3060

gctgtgttca cccagtttgt actcgcaggt ggtttattaa aatcatttgc aatatcaggt 3120

aatgagacgt tcaaaaccat ttcatttaat acgctaaaaa aagataaaat gcaaagccaa 3180

attaaaattt ggttgtgtcg taaattcgat tgtgaatagg atgtattcac atttcaccct 3240

ccaataatga gggcagacgt agtttatagg gttaatgata cgcttccctc ttttaattga 3300

accctgttac attcattatt cattacactt cataattaat tcctcctaaa cttgattaaa 3360

acattttacc acatataaac taagttttaa attcagtatt tcatcactta tacaacaata 3420

tggcccgttt gttgaactac tctttaataa aataattttt ccgttcccaa ttccacattg 3480

caataataga aaatccatct tcatcggctt tttcgtcatc atctgtatga atcaaatcgc 3540

cttcttctgt gtcatcaagg tttaattttt tatgtatttc ttttaacaaa ccaccatagg 3600

agattaacct tttacggtgt aaaccttcct ccaaatcaga caaacgtttc aaattctttt 3660

cttcatcatc ggtcataaaa tccgtatcct ttacaggata ttttgcagtt tcgtcaattg 3720

ccgattgtat atccgattta tatttatttt tcggtcgaat catttgaact tttacatttg 3780

gatcatagtc taatttcatt gcctttttcc aaaattgaat ccattgtttt tgattcacgt 3840

agttttctgt attcttaaaa taagttggtt ccacacatac caatacatgc atgtgctgat 3900

tataagaatt atctttatta tttattgtca cttccgttgc acgcataaaa ccaacaagat 3960

ttttattaat ttttttatat tgcatcattc ggcgaaatcc ttgagccata tctgacaaac 4020

tcttatttaa ttcttcgcca tcataaacat ttttaactgt taatgtgaga aacaaccaac 4080

gaactgttgg cttttgttta ataacttcag caacaacctt ttgtgactga atgccatgtt 4140

tcattgctct cctccagttg cacattggac aaagcctgga tttacaaaac cacactcgat 4200

acaactttct ttcgcctgtt tcacgatttt gtttatactc taatatttca gcacaatctt 4260

ttactctttc agccttttta aattcaagaa tatgcagaag ttcaaagtaa tcaacattag 4320

cgattttctt ttctctccat ggtctcactt ttccactttt tgtcttgtcc actaaaaccc 4380

ttgatttttc atctgaataa atgctactat taggacacat aatattaaaa gaaaccccca 4440

tctatttagt tatttgtttg gtcacttata actttaacag atggggtttt tctgtgcaac 4500

caattttaag ggttttccaa tactttaaaa cacatacata ccaacacttc aacgcacctt 4560

tcagcaacta aaataaaaat gacgttattt ctatatgtat caagataaga aagaacaagt 4620

tcaaaaccat caaaaaaaga caccttttca ggtgcttttt ttattttata aactcattcc 4680

ctgatctcga cttcgttctt tttttacctc tcggttatga gttagttcaa attcgttctt 4740

tttaggttct aaatcgtgtt tttcttggaa ttgtgctgtt ttatccttta ccttgtctac 4800

aaacccctta aaaacgtttt taaaggcttt taagcgtctg tacgttcctt aaggaattat 4860

tccttagtgc tttctaggtt aatgtcatga taataatggt ttcttagacg tcaggtggca 4920

cttttcgggg aaatgtccgc ggaaccccta tttgtttatt tttctaaata cattcaaata 4980

tgtatccgct catgagacaa taaccctgat aaatgcttca ataatattga aaaaggaaga 5040

gtatgagtat tcaacatttc cgtgtcgccc ttattccctt ttttgcggca ttttgccttc 5100

ctgtttttgc tcacccagaa acgctggtga aagtaaaaga tgctgaagat cagttgggtg 5160

cacgagtggg ttacatcgaa ctggatctca acagcggtaa gatccttgag agttttcgcc 5220

ccgaagaacg ttttccaatg atgagcactt ttaaagttct gctatgtggc gcggtattat 5280

cccgtgttga cgccgggcaa gagcaactcg gtcgccgcat acactattct cagaatgact 5340

tggttgagta ctcaccagtc acagaaaagc atcttacgga tggcatgaca gtaagagaat 5400

tatgcagtgc tgccataacc atgagtgata acactgcggc caacttactt ctgacaacga 5460

tcggaggacc gaaggagcta accgcttttt tgcacaacat gggggatcat gtaactcgcc 5520

ttgatcgttg ggaaccggag ctgaatgaag ccataccaaa cgacgagcgt gacaccacga 5580

tgcctgcagc aatggcaaca acgttgcgca aactattaac tggcgaacta cttactctag 5640

cttcccggca acaattaata gactggatgg aggcggataa agttgcagga ccacttctgc 5700

gctcggccct tccggctggc tggtttattg ctgataaatc tggagccggt gagcgtgggt 5760

ctcgcggtat cattgcagca ctggggccag atggtaagcc ctcccgtatc gtagttatct 5820

acacgacggg gagtcaggca actatggatg aacgaaatag acagatcgct gagataggtg 5880

cctcactgat taagcattgg taactgtcag accaagttta ctcatatata ctttagattg 5940

atttaaaact tcatttttaa tttaaaagga tctaggtgaa gatccttttt gataatctca 6000

tgaccaaaat cccttaacgt gagttttcgt tccactgagc gtcagacccc ttaataagat 6060

gatcttcttg agatcgtttt ggtctgcgcg taatctcttg ctctgaaaac gaaaaaaccg 6120

ccttgcaggg aggtttttcg aaggttctct gagctaccaa ctctttgaac cgaggtaact 6180

ggcttgcagg agcgcagtca ccaaaacttg tcctttcagt ttagccttaa ccggcgcatg 6240

acttcaagac taactcctct aaatcaatta ccagtggctg ctgccagtgg tgcttttgca 6300

tgtctttccg ggttggactc aagacgatag ttaccggata aggcgcagcg gtcggactga 6360

acggggggtt cgtgcataca gtccagcttg gagcgaactg cctacccgga actgagtgtc 6420

aggcgtggaa tgagacaaac gcggccataa cagcggaatg acaccggtaa accgaaaggc 6480

aggaacagga gagcgcacga gggagccgcc agggggaaac gcctggtatc tttatagtcc 6540

tgtcgggttt cgccaccact gatttgagcg tcagatttcg tgatgcttgt caggggggcg 6600

gagcctatgg aaaaacggct ttgccc 6626

<210> 31
<211> 248
<212> DNA
<213> Artificial sequence

<220>
<223> CpIa promoter insert

<400>  31
catatgactc cctcctaatt caagtttatt taagattcaa tcttcaaata atgaaaatat      60

attttaaatt tttcttttta acataagata acctcctttc tattttgaat ttagttataa     120

tattatgtat taatattata taaatattat acagtatatg acaaagaaaa tctctatata     180

aatacaaaaa atatattttt atcaataaaa gtaatttaat gtaaaattag attgtatttt     240

ctgaattc                                                              248


<210>  32
<211>  37
<212>  DNA
<213>  Artificial sequence

<220>
<223>  MCS for pCIP

<400>  32
aagcttctag agatctgcag gtcgacggat ccccggg                               37


<210>  33
<211>  5115
<212>  DNA
<213>  Artificial sequence

<220>
<223>  pCIP

<400>  33
aagcttctag agatctgcag gtcgacggat ccccgggcat atgactccct cctaattcaa      60

gtttatttaa gattcaatct tcaaataatg aaaatatatt ttaaattttt ctttttaaca     120

taagataacc tcctttctat tttgaattta gttataatat tatgtattaa tattatataa     180

atattataca gtatatgaca aagaaaatct ctatataaat acaaaaaata tatttttatc     240

aataaaagta atttaatgta aaattagatt gtattttctg aattcctgtt ataaaaaaag     300

gatcaatttt gaactctctc ccaaagttga tcccttaacg atttagaaat ccctttgaga     360

atgtttatat acattcaagg taaccagcca actaatgaca atgattcctg aaaaaagtaa     420

taacaaatta ctatacagat aagttgactg atcaacttcc ataggtaaca acctttgatc     480

aagtaagggt atggataata aaccacctac aattgcaata cctgttccct ctgataaaaa     540

gctggtaaag ttaagcaaac tcattccagc accagcttcc tgctgtttca agctacttga     600

aacaattgtt gatataactg ttttggtgaa cgaaagccca cctaaaacaa atacgattat     660

aattgtcatg aaccatgatg ttgtttctaa aagaaaggaa gcagttaaaa agctaacaga     720

aagaaatgta actccgatgt ttaacacgta taaaggacct cttctatcaa caagtatccc     780

accaatgtag ccgaaaataa tgacactcat tgttccaggg aaaataatta cacttccgat     840

ttcggcagta cttagctggt gaacatcttt catcatataa ggaaccatag agacaaaccc 900
tgctactgtt ccaaatataa ttcccccaca aagaactcca atcataaaag gtatattttt 960
ccctaatccg ggatcaacaa aaggatctgt tactttcctg atatgtttta caaatatcag 1020
gaatgacagc acgctaacga taagaaaaga aatgctatat gatgttgtaa acaacataaa 1080
aaatacaatg cctacagaca ttagtataat tcctttgata tcaaaatgac cttttatcct 1140
tacttctttc tttaataatt tcataagaaa cggaacagtg ataattgtta tcataggaat 1200
gagtagaaga taggaccaat gaatataatg ggctatcatt ccaccaatcg ctggaccgac 1260
tccttctccc atggctacta tcgatccaat aagaccaaat gctttacccc tattttcctt 1320
tggaatatag cgcgcaacta caaccattac gagtgctgga aatgcagctg caccagcccc 1380
ttgaataaaa cgagccataa taagtaagga aaagaaagaa tggccaacaa acccaattac 1440
cgacccgaaa caatttatta taattccaaa taggagtaac cttttgatgc ctaattgatc 1500
agatagcttt ccatatacag ctgttccaat ggaaaaggtt aacataaagg ctgtgttcac 1560
ccagtttgta ctcgcaggtg gtttattaaa atcatttgca atatcaggta atgagacgtt 1620
caaaaccatt tcatttaata cgctaaaaaa agataaaatg caaagccaaa ttaaaatttg 1680
gttgtgtcgt aaattcgatt gtgaatagga tgtattcaca tttcaccctc caataatgag 1740
ggcagacgta gtttataggg ttaatgatac gcttccctct tttaattgaa ccctgttaca 1800
ttcattattc attacacttc ataattaatt cctcctaaac ttgattaaaa catttaccca 1860
catataaact aagttttaaa ttcagtattt catcacttat acaacaatat ggcccgtttg 1920
ttgaactact ctttaataaa ataatttttc cgttcccaat tccacattgc aataatagaa 1980
aatccatctt catcggcttt ttcgtcatca tctgtatgaa tcaaatcgcc ttcttctgtg 2040
tcatcaaggt ttaatttttt atgtatttct tttaacaaac caccatagga gattaacctt 2100
ttacggtgta aaccttcctc caaatcagac aaacgtttca aattcttttc ttcatcatcg 2160
gtcataaaat ccgtatcctt tacaggatat tttgcagttt cgtcaattgc cgattgtata 2220
tccgatttat atttattttt cggtcgaatc atttgaactt ttacatttgg atcatagtct 2280
aatttcattg cctttttcca aaattgaatc cattgttttt gattcacgta gttttctgta 2340
ttcttaaaat aagttggttc cacacatacc aatacatgca tgtgctgatt ataagaatta 2400
tctttattat ttattgtcac ttccgttgca cgcataaaac caacaagatt tttattaatt 2460
tttttatatt gcatcattcg gcgaaatcct tgagccatat ctgacaaact cttatttaat 2520
tcttcgccat cataaacatt tttaactgtt aatgtgagaa acaaccaacg aactgttggc 2580
ttttgtttaa taacttcagc aacaaccttt tgtgactgaa tgccatgttt cattgctctc 2640

ctccagttgc acattggaca aagcctggat ttacaaaacc acactcgata caactttctt 2700

tcgcctgttt cacgattttg tttatactct aatatttcag cacaatcttt tactctttca 2760

gcctttttaa attcaagaat atgcagaagt tcaaagtaat caacattagc gattttcttt 2820

tctctccatg gtctcacttt tccacttttt gtcttgtcca ctaaaaccct tgatttttca 2880

tctgaataaa tgctactatt aggacacata atattaaaag aaacccccat ctatttagtt 2940

atttgtttgg tcacttataa ctttaacaga tggggttttt ctgtgcaacc aattttaagg 3000

gttttccaat actttaaaac acatacatac caacacttca acgcaccttt cagcaactaa 3060

aataaaaatg acgttatttc tatatgtatc aagataagaa agaacaagtt caaaaccatc 3120

aaaaaaagac accttttcag gtgctttttt tattttataa actcattccc tgatctcgac 3180

ttcgttcttt ttttacctct cggttatgag ttagttcaaa ttcgttcttt ttaggttcta 3240

aatcgtgttt ttcttggaat tgtgctgttt tatcctttac cttgtctaca aaccccttaa 3300

aaacgttttt aaaggctttt aagcgtctgt acgttcctta aggaattatt ccttagtgct 3360

ttctaggtta atgtcatgat aataatggtt tcttagacgt caggtggcac ttttcgggga 3420

aatgtccgcg gaacccctat ttgtttattt ttctaaatac attcaaatat gtatccgctc 3480

atgagacaat aaccctgata aatgcttcaa taatattgaa aaaggaagag tatgagtatt 3540

caacatttcc gtgtcgccct tattcccttt tttgcggcat tttgccttcc tgtttttgct 3600

cacccagaaa cgctggtgaa agtaaaagat gctgaagatc agttgggtgc acgagtgggt 3660

tacatcgaac tggatctcaa cagcggtaag atccttgaga gttttcgccc cgaagaacgt 3720

tttccaatga tgagcacttt taaagttctg ctatgtggcg cggtattatc ccgtgttgac 3780

gccgggcaag agcaactcgg tcgccgcata cactattctc agaatgactt ggttgagtac 3840

tcaccagtca cagaaaagca tcttacggat ggcatgacag taagagaatt atgcagtgct 3900

gccataacca tgagtgataa cactgcggcc aacttacttc tgacaacgat cggaggaccg 3960

aaggagctaa ccgctttttt gcacaacatg ggggatcatg taactcgcct tgatcgttgg 4020

gaaccggagc tgaatgaagc cataccaaac gacgagcgtg acaccacgat gcctgcagca 4080

atggcaacaa cgttgcgcaa actattaact ggcgaactac ttactctagc ttcccggcaa 4140

caattaatag actggatgga ggcggataaa gttgcaggac cacttctgcg ctcggccctt 4200

ccggctggct ggtttattgc tgataaatct ggagccggtg agcgtgggtc tcgcggtatc 4260

attgcagcac tggggccaga tggtaagccc tcccgtatcg tagttatcta cacgacgggg 4320

agtcaggcaa ctatggatga acgaaataga cagatcgctg agataggtgc ctcactgatt 4380

aagcattggt aactgtcaga ccaagtttac tcatatatac tttagattga tttaaaactt 4440

catttttaat ttaaaaggat ctaggtgaag atcctttttg ataatctcat gaccaaaatc 4500

ccttaacgtg agttttcgtt ccactgagcg tcagaccccg taataagatg atcttcttga 4560

gatcgttttg gtctgcgcgt aatctcttgc tctgaaaacg aaaaaaccgc cttgcaggga 4620

ggtttttcga aggttctctg agctaccaac tctttgaacc gaggtaactg gcttgcagga 4680

gcgcagtcac caaaacttgt cctttcagtt tagccttaac cggcgcatga cttcaagact 4740

aactcctcta aatcaattac cagtggctgc tgccagtggt gcttttgcat gtctttccgg 4800

gttggactca agacgatagt taccggataa ggcgcagcgg tcggactgaa cggggggttc 4860

gtgcatacag tccagcttgg agcgaactgc ctacccggaa ctgagtgtca ggcgtggaat 4920

gagacaaacg cggccataac agcggaatga caccggtaaa ccgaaaggca ggaacaggag 4980

agcgcacgag ggagccgcca gggggaaacg cctggtatct ttatagtcct gtcgggtttc 5040

gccaccactg atttgagcgt cagatttcgt gatgcttgtc aggggggcgg agcctatgga 5100

aaaacggctt tgccc 5115


<210> 34
<211> 21
<212> DNA
<213> Artificial sequence

<220>
<223> Forward primer for pHY300PLK-CpA

<400> 34
tttaaaaaat attcaaaaaa t 21


<210> 35
<211> 23
<212> DNA
<213> Artificial sequence

<220>
<223> Reverse primer for pHY300PLK-CpA

<400> 35
ggaagctttt attttgtaaa tac 23


<210> 36
<211> 1385
<212> DNA
<213> Clostridium perfringens

<400> 36
aaaaatattt taaaaaatat tcaaaaattt agtgaggtta tggtaattat atggtataat 60

ttcagtgcaa gtgttaatcg ttatcaaaaa aggggagatt aatacttgaa aaaaaataac 120

gggggatata aaaatgaaaa gaaagatttg taaggcgctt atttgtgcta cgctagcaac 180

tagcctatgg gctggggcat caactaaagt ctacgcttgg gatggaaaga ttgatggaac 240

aggaactcat gctatgattg taactcaagg ggtttcaatc ttagaaaatg atctgtccaa 300

aaatgaacca gaaagtgtaa gaaaaaactt agagatttta aaagagaaca tgcatgagct 360

tcaattaggt tctacttatc cagattatga taagaatgca tatgatctat atcaagatca 420

tttctgggat cctgatacag ataataattt ctcaaaggat aatagttggt atttagctta 480

ttctatacct gacacagggg aatcacaaat aagaaaattt tcagcattag ctagatatga 540

atggcaaaga ggaaactata aacaagctac attctatctt ggagaggcta tgcactattt 600

tggagatata gatactccat atcatcctgc taatgttact gccgttgata gcgcaggaca 660

tgttaagttt gagacttttg cagaggaaag aaaagaacag tataaaataa acacagcagg 720

ttgcaaaact aatgaggatt tttatgctga tatcttaaaa aacaaagatt ttaatgcatg 780

gtcaaaagaa tatgcaagag gttttgctaa aacaggaaaa tcaatatact atagtcatgc 840

tagcatgagt catagttggg atgattggga ttatgcagca aaggtaactt tagctaactc 900

tcaaaaagga acagcaggat atatttatag attcttacac gatgtatcag agggtaatga 960

tccatcagtt ggaaagaatg taaaagaact agtagcttac atatcaacta gtggtgagaa 1020

agatgctgga acagatgact acatgtattt tggaatcaaa acaaaggatg gaaaaactca 1080

agaatgggaa atggacaacc caggaaatga ttttatgact ggaagtaaag acacttatac 1140

tttcaaatta aaagatgaaa atctaaaaat tgatgatata caaaatatgt ggattagaaa 1200

aagaaaatat acagcattcc cagatgctta taagccagaa aacataaaga taatagcaaa 1260

tggaaaagtt gtagtagaca aagatataaa cgagtggatt tcaggaaatt caacttataa 1320

tataaaataa taaaagtaaa aaaataatta ttggttttgg tggtatttac aaaataaaag 1380

cttcc 1385

<210> 37
<211> 20
<212> DNA
<213> Artificial sequence

<220>
<223> Forward primer for CpB2

<400> 37
ttagataaaa gtgtaaaaga 20

<210> 38
<211> 19
<212> DNA

<213>  Artificial sequence

<220>
<223>  Reverse primer for CpB2

<400>  38
ttaggttttt atataataa                                                    19


<210>  39
<211>  960
<212>  DNA
<213>  Clostridium perfringens

<400>  39
ttagataaaa gtgtaaaaga attattttta ttttaaattt gttaaaattt tgatataatt        60

gaattgtaaa aaaaatttca ggggggaata taaatgaaaa aaattatttc aaagtttact       120

gtaattttta tgttttcata ttttcttatt gttggagcaa taagtccaat gaaagcaagt       180

gcaaaggaaa tcgacgctta tagaaaggta atggagaatt atcttaatgc ttttaaaaac       240

tacgatatta atacgattgt aaacgtatca gaagatgaaa gagtgaatag tgatgaaaag       300

tataaagaga tgttagaaga gtttaaatat gatcctaacc aacaactaaa atcttttgaa       360

atacttaatt cacaaaagat tgataataaa gaaatattta atgtaaaaac tgaatttatg       420

aatggtgcaa tttatgatat gaaatttact gtatcatcta aagatgggga attaatagta       480

tctgacatgg aaagaacaaa aattgagaat gagggaaaat atattttaac accatcattt       540

agaactcaag tttgtacatg ggatgatgaa ttatcacaat caattggggg agttgatcca       600

aaaacatatt ctactagatt tacatattat gcagacaata tattattaaa ctttagacaa       660

tatgcaactt caggttcaag agatttaaaa gtagaatata gtgttgtaga tcattggtta       720

tggggagatg atgttaaagc ttctcaaatg gtgtatggtc aaaaccctga ttctgctaga       780

caaataagat tatatataga aaaaggacaa tctttctata aatatagaat aagaatacaa       840

aactttacac ctgcatcaat tagagtattt ggtgaaggat attgtgcata gaaaaaaata       900

tgaagtgact aagtcacttc atattttttt tactattaat tttattatat aaaaacctaa       960


<210>  40
<211>  18
<212>  DNA
<213>  Artificial sequence

<220>
<223>  Forward primer for BcSMase

<400>  40
atggaggtat ggaacgtg                                                     18

<210>  41
<211>  18
<212>  DNA
<213>  Artificial sequence

<220>
<223>  Reverse primer for BcSMase

<400>  41
ctacttcata gaaatagt                                                    18


<210>  42
<211>  1017
<212>  DNA
<213>  Bacillus cereus

<400>  42
atggaggtat ggaacgtgaa atgtaaattg ctaaaaggtg tacttagctt aggtgttggt      60

ttaggagctt tatatagcgg aacgtcagct caagcagaag tgtctacaaa tcaaaatgat     120

acattaaaag tgatgacgca taatgtatat atgctatcaa caaacttata tccgaactgg     180

ggacaaactg agcgtgctga tttaatcggg gcggcagatt atataaagaa tcaagatgta     240

gttatattaa atgaagtgtt tgataatagc gcttcagatc gtttattagg gaatttgaag     300

aaagaatatc caaatcaaac agcagtatta ggtcgtagta gtggaagtga atgggataaa     360

acgttaggaa actattcatc ttcaactcct gaagatggtg gcgttgcgat tgtgagcaaa     420

tggccaatcg ctgaaaagat tcaatatgta tttgcaaaag gatgcgggcc agataattta     480

tcgaataaag gatttgtata cacgaaaatt aagaaaaatg atcgcttcgt tcacgtgatt     540

ggtacacatt tgcaagcgga agatagtatg tgcggaaaaa cttcacctgc atctgtacgt     600

acagaccaat taaaagaaat tcaagacttt attaaaaata aaaatatacc aaataatgag     660

tatgtgttaa ttggcggtga tatgaacgta aataaaatta atgcagagaa caagaatgat     720

tcagagtatg catctatgtt taaaacattg aacgcttctg taccatctta tactggacat     780

acagcgactt gggacgcaac gacaaacagt attgcaaaat ataatttccc tgatagtcct     840

gctgagtatt tagattatat tattgcaagt aaagaccatg cgaacccatc atatatagag     900

aataaggtgt tacagccgaa atctccacaa tggactgtta catcgtggtt ccaaaaatat     960

acgtataatg attactctga tcattatcca gtagaggcga ctatttctat gaagtag       1017


<210>  43
<211>  30
<212>  DNA
<213>  Artificial sequence

<220>
<223>  Forward primer for pCIP-CpA

<400>  43
gggcatatga tgaaaagaaa gatttgtaag                                        30


<210>  44
<211>  30
<212>  DNA
<213>  Artificial sequence

<220>
<223>  Reverse primer for pCIP-CpA

<400>  44
ccctctagat tattttatat tataagttga                                        30


<210>  45
<211>  1212
<212>  DNA
<213>  Artificial sequence

<220>
<223>  Insert for pCIP-CpA

<400>  45
gggcatatga aaagaaagat ttgtaaggcg cttatttgtg ccgcgctagc aactagccta       60

tgggctgggg catcaactaa agtctacgct tgggatggaa agattgatgg aacaggaact      120

catgctatga ttgtaactca aggggtttca atcttagaaa atgatctgtc caaaaatgaa      180

ccagaaagtg taagaaaaaa cttagagatt ttaaaagaga acatgcatga gcttcaatta      240

ggttctactt atccagatta tgataagaat gcatatgatc tatatcaaga tcatttctgg      300

gatcctgata cagataataa tttctcaaag gataatagtt ggtatttagc ttattctata      360

cctgacacag gggaatcaca aataagaaaa ttttcagcat tagctagata tgaatggcaa      420

agaggaaact ataaacaagc tacattctat cttggagagg ctatgcacta ttttggagat      480

atagatactc catatcatcc tgctaatgtt actgccgttg atagcgcagg acatgttaag      540

tttgagactt ttgcagagga aagaaaagaa cagtataaaa taaacacagc aggttgcaaa      600

actaatgagg ctttttatac tgatatctta aaaaacaaag attttaatgc atggtcaaaa      660

gaatatgcaa gaggttttgc taaaacagga aaatcaatat actatagtca tgctagcatg      720

agtcatagtt gggatgattg ggattatgca gcaaaggtaa ctttagctaa ctctcaaaaa      780

ggaacagcgg gatatattta tagattctta cacgatgtat cagagggtaa tgatccatca      840

gttggaaaga atgtaaaaga actagtagct tacatatcaa ctagtggtga gaaagatgct      900

ggaacagatg actacatgta ttttggaatc aaaacaaagg atggaaaaac tcaagaatgg      960

gaaatggaca acccaggaaa tgattttatg actggaagta aagacactta tactttcaaa     1020

ttaaaagatg aaaatctaaa aattgatgat atacaaaata tgtggattag aaaaagaaaa 1080

tatacagcat tctcagatgc ttataagcca gaaaacataa agataatagc aaatggaaaa 1140

gttgtagtgg acaaagatat aaacgagtgg atttcaggaa attcaactta taatataaaa 1200

taatctagag gg 1212

<210> 46
<211> 30
<212> DNA
<213> Artificial sequence

<220>
<223> Forward primer for pCIP-CpB2

<400> 46
aggcatatga aaaaaattat ttcaaagttt 30

<210> 47
<211> 26
<212> DNA
<213> Artificial sequence

<220>
<223> Reverse primer for pCIP-CpB2

<400> 47
cctctagact atgcacaata tccttc 26

<210> 48
<211> 812
<212> DNA
<213> Artificial sequence

<220>
<223> Insert for pCIP-CpB2

<400> 48
aggcatatga aaaaaattat ttcaaagttt actgtaattt ttatgttttc atattttctt 60

attgttggag caataagtcc aatgaaagca agtgcaaagg aaatcgacgc ttatagaaag 120

gtaatggaga attatcttaa tgcttttaaa aactacgata ttaatacgat tgtaaacgta 180

tcagaagatg aaagagtgaa tagtgatgaa aagtataaag agatgttaga agagtttaaa 240

tatgatccta accaacaact aaaatctttt gaaatactta attcacaaaa gattgataat 300

aaagaaatat ttaatgtaaa aactgaattt atgaatggtg caatttatga tatgaaattt 360

actgtatcat ctaaagatgg ggaattaata gtatctgaca tggaaagaac aaaaattgag 420

aatgagggaa aatatatttt aacaccatca tttagaactc aagtttgtac atgggatgat 480

gaattatcac aatcaattgg gggagttgat ccaaaaacat attctactag atttacatat 540

tatgcagaca atatattatt aaactttaga caatatgcaa cttcaggttc aagagattta 600

aaagtagaat atagtgttgt agatcattgg ttatggggag atgatgttaa agcttctcaa 660

atggtgtatg gtcaaaaccc tgattctgct agacaaataa gattatatat agaaaaagga 720

caatctttct ataaatatag aataagaata caaaacttta cacctgcatc aattagagta 780

tttggtgaag gatattgtgc atagtctaga gg 812

<210> 49
<211> 24
<212> DNA
<213> Artificial sequence

<220>
<223> Forward primer for pCIP-BcSMase

<400> 49
catatgatgg aggtatggaa cgtg 24

<210> 50
<211> 24
<212> DNA
<213> Artificial sequence

<220>
<223> Reverse primer for pCIP-BcSMase

<400> 50
tctagactac ttcatagaaa tagt 24

<210> 51
<211> 1029
<212> DNA
<213> Artificial sequence

<220>
<223> Insert for pCIP-BcSMase

<400> 51
catatgatgg aggtatggaa cgtgaaatgt aaattgctaa aaggtgtact tagcttaggt 60

gttggtttag gagctttata tagcggaacg tcagctcaag cagaagtgtc tacaaatcaa 120

aatgatacat taaaagtgat gacgcataat gtatatatgc tatcaacaaa cttatatccg 180

aactggggac aaactgagcg tgctgattta atcggggcgg cagattatat aaagaatcaa 240

gatgtagtta tattaaatga agtgtttgat aatagcgctt cagatcgttt attagggaat 300

ttgaagaaag aatatccaaa tcaaacagca gtattaggtc gtagtagtgg aagtgaatgg 360

gataaaacgt taggaaacta ttcatcttca actcctgaag atggtggcgt tgcgattgtg 420

agcaaatggc caatcgctga aaagattcaa tatgtatttg caaaaggatg cgggccagat 480

aatttatcga ataaaggatt tgtatacacg aaaattaaga aaaatgatcg cttcgttcac 540

gtgattggta cacatttgca agcggaagat agtatgtgcg gaaaaacttc acctgcatct 600

gtacgtacag accaattaaa agaaattcaa gactttatta aaaataaaaa tataccaaat 660

aatgagtatg tgttaattgg cggtgatatg aacgtaaata aaattaatgc agagaacaag 720

aatgattcag agtatgcatc tatgtttaaa acattgaacg cttctgtacc atcttatact 780

ggacatacag cgacttggga cgcaacgaca aacagtattg caaaatataa tttccctgat 840

agtcctgctg agtatttaga ttatattatt gcaagtaaag accatgcgaa cccatcatat 900

atagagaata aggtgttaca gccgaaatct ccacaatgga ctgttacatc gtggttccaa 960

aaatatacgt ataatgatta ctctgatcat tatccagtag aggcgactat ttctatgaag 1020

tagtctaga 1029

<210> 52
<211> 30
<212> DNA
<213> Artificial sequence

<220>
<223> Forward primer for pCIP-CbPLC

<400> 52
catatgatga ataagaaaaa aatattaaaa 30

<210> 53
<211> 30
<212> DNA
<213> Artificial sequence

<220>
<223> Reverse primer for pCIP-CbPLC

<400> 53
tctagattat ttattattta tatagaatgt 30

<210> 54
<211> 1212
<212> DNA
<213> Artificial sequence

<220>
<223> Insert for pCIP-CbPLC

<400> 54
catatgatga ataagaaaaa aatattaaaa tttatttgta gtgcagtatt atcattcaca 60

ttattttcag gctataaaag ttatgcatgg gatggaaaac ctgatggtac aggaactcat 120

gcagtaatag ttacacaagc tgtagaaatg ttaaaaaatg atgttataag tacatcacct 180

ttaagtgtaa aagaaaattt taaaatttta gaatctaatt taaaaaaatt acaacatggt 240

tctacttatc cagattatga tccaaaggca tatgcattat atcaagatca tttttgggat 300

cctgatacag ataataattt tactaaagat agtaaatggt atctatcata tgcaattagt 360

caaacaggag aatctcaact tagaaagtta tttgcattag ctaaagatga gtggaaaaaa 420

ggaaattatg aacaagcaac atggctttta ggacaaggtt tacattactt tggagatttc 480

aatactcctt atcatccatc taatgttaca gctgtagata gtacaggtca tgtgaaattt 540

gaaacttatg ttgaagaaag aaaagattca tataaattaa attcagcagg aactaatagt 600

gtaaaggaat tttatctaac tacattacaa aatactaatc ttgataattg gataacagag 660

tattctagag gttgggctaa aaaagctaag aatatgtatt atgctcatgc tactatgaat 720

cataattgga aagattggga aatagcagct aatgaaacta tgcataatgt tcaaataggt 780

agtgctggaa taatatacag attccttaat gaagtatcag gaacaataaa tacaactgaa 840

aattctaaaa taaatgaaat aatgatagta ataaaaactg cagatgaaga taaagcaggt 900

acagatcatt ctattcattt cggaattgaa gcaaaagatg gaaagaagta tgaatggact 960

cttgataatc caggtaatga ttttgaaaaa aatcaagaag atagttatag aattaattta 1020

aaagataata aattaacact tcaagatata gctaaaacat ggataagaaa agaaagaggc 1080

gctggagttc aagatgattg gaaacctgaa tatgtaaaag taattataaa ttcagatgtt 1140

aagtatcaag ctaatattaa tgagtggttt ggagataaca aaacattcta tataaataat 1200

aaataatcta ga 1212

<210> 55
<211> 30
<212> DNA
<213> Artificial sequence

<220>
<223> Forward primer for pCIP-CbC3

<400> 55
aggcatatga aaaaaattat ttcaaagttt 30

<210> 56
<211> 29
<212> DNA
<213> Artificial sequence

<220>
<223> Reverse primer for pCIP-CbC3

<400> 56
tcctctagag aagtgactta gtcacttca 29

<210> 57
<211> 769
<212> DNA
<213> Artificial sequence

<220>
<223> Insert for pCIP-CbC3

<400> 57
ggcatatgaa aggtttaaga aaatcaattt tatgtttagt tttgtcagca ggagtaatag 60

ctccagtaac atctgggatg attcaaagtc ctcaaaaatg ttatgcttat tccattaatc 120

aaaaggctta ttcaaatact taccaggagt ttactaatat tgatcaagca aaagcttggg 180

gtaatgctca gtataaaaag tatggactaa gcaaatcaga aaaagaagct atagtatcat 240

atactaaaag cgctagtgaa ataaatggaa agctaagaca aaataaggga gttatcaatg 300

gatttccttc aaatttaata aaacaagttg aacttttaga taaatctttt aataaaatga 360

agacccctga aaatattatg ttatttagag gcgacgaccc tgcttattta ggaacagaat 420

ttcaaaacac tcttcttaat tcaaatggta caattaataa aacggctttt gaaaaggcta 480

aagctaagtt tttaaataaa gatagacttg aatatggata tattagtact tcattaatga 540

atgtttctca atttgcagga agaccaatta ttacaaaatt taaagtagca aaaggctcaa 600

aggcaggata tattgaccct attagtgctt ttgcaggaca acttgaaatg ttgcttccta 660

gacatagtac ttatcatata gacgatatga gattgtcttc tgatggtaaa caaataataa 720

ttacagcaac aatgatgggc acagctatca atcctaaata atctagagg 769


<210> 58
<211> 6283
<212> DNA
<213> Artificial sequence

<220>
<223> pCIP-CpA

<400> 58
aagcttctag attattttat attataagtt gaatttcctg aaatccactc gtttatatct 60

ttgtccacta caacttttcc atttgctatt atctttatgt tttctggctt ataagcatct 120

gagaatgctg tatattttct ttttctaatc cacatatttt gtatatcatc aatttttaga 180

ttttcatctt ttaatttgaa agtataagtg tctttacttc cagtcataaa atcatttcct 240

gggttgtcca tttcccattc ttgagttttt ccatcctttg ttttgattcc aaaatacatg 300

tagtcatctg ttccagcatc tttctcacca ctagttgata tgtaagctac tagttctttt 360

acattctttc caactgatgg atcattaccc tctgatacat cgtgtaagaa tctataaata 420

tatcccgctg ttcctttttg agagttagct aaagttacct ttgctgcata atcccaatca 480

tcccaactat gactcatgct agcatgacta tagtatattg attttcctgt tttagcaaaa 540

cctcttgcat attcttttga ccatgcatta aaatctttgt tttttaagat atcagtataa 600

aaagcctcat tagttttgca acctgctgtg tttattttat actgttcttt tctttcctct 660

gcaaaagtct caaacttaac atgtcctgcg ctatcaacgg cagtaacatt agcaggatga 720

tatggagtat ctatatctcc aaaatagtgc atagcctctc caagatagaa tgtagcttgt 780

ttatagtttc ctctttgcca ttcatatcta gctaatgctg aaaattttct tatttgtgat 840

tcccctgtgt caggtataga ataagctaaa taccaactat tatcctttga gaaattatta 900

tctgtatcag gatcccagaa atgatcttga tatagatcat atgcattctt atcataatct 960

ggataagtag aacctaattg aagctcatgc atgttctctt ttaaaatctc taagtttttt 1020

cttacacttt ctggttcatt tttggacaga tcattttcta agattgaaac cccttgagtt 1080

acaatcatag catgagttcc tgttccatca atctttccat cccaagcgta gactttagtt 1140

gatgccccag cccataggct agttgctagc gcggcacaaa taagcgcctt acaaatcttt 1200

cttttcatat gactccctcc taattcaagt ttatttaaga ttcaatcttc aaataatgaa 1260

aatatatttt aaatttttct ttttaacata agataacctc ctttctattt tgaatttagt 1320

tataatatta tgtattaata ttatataaat attatacagt atatgacaaa gaaaatctct 1380

atataaatac aaaaaatata tttttatcaa taaaagtaat ttaatgtaaa attagattgt 1440

attttctgaa ttcctgttat aaaaaaagga tcaattttga actctctccc aaagttgatc 1500

ccttaacgat ttagaaatcc ctttgagaat gtttatatac attcaaggta accagccaac 1560

taatgacaat gattcctgaa aaaagtaata acaaattact atacagataa gttgactgat 1620

caacttccat aggtaacaac ctttgatcaa gtaagggtat ggataataaa ccacctacaa 1680

ttgcaatacc tgttccctct gataaaaagc tggtaaagtt aagcaaactc attccagcac 1740

cagcttcctg ctgtttcaag ctacttgaaa caattgttga tataactgtt ttggtgaacg 1800

aaagcccacc taaaacaaat acgattataa ttgtcatgaa ccatgatgtt gtttctaaaa 1860

gaaaggaagc agttaaaaag ctaacagaaa gaaatgtaac tccgatgttt aacacgtata 1920

aaggacctct tctatcaaca agtatcccac caatgtagcc gaaaataatg acactcattg 1980

ttccagggaa aataattaca cttccgattt cggcagtact tagctggtga acatctttca 2040

tcatataagg aaccatagag acaaaccctg ctactgttcc aaatataatt cccccacaaa 2100

gaactccaat cataaaaggt atatttttcc ctaatccggg atcaacaaaa ggatctgtta 2160

ctttcctgat atgttttaca aatatcagga atgacagcac gctaacgata agaaaagaaa 2220

tgctatatga tgttgtaaac aacataaaaa atacaatgcc tacagacatt agtataattc 2280

ctttgatatc aaaatgacct tttatcctta cttctttctt taataatttc ataagaaacg 2340

gaacagtgat aattgttatc ataggaatga gtagaagata ggaccaatga atataatggg 2400

ctatcattcc accaatcgct ggaccgactc cttctcccat ggctactatc gatccaataa 2460

gaccaaatgc tttaccccta ttttcctttg gaatatagcg cgcaactaca accattacga 2520

gtgctggaaa tgcagctgca ccagcccctt gaataaaacg agccataata agtaaggaaa 2580

agaaagaatg gccaacaaac ccaattaccg acccgaaaca atttattata attccaaata 2640

ggagtaacct tttgatgcct aattgatcag atagctttcc atatacagct gttccaatgg 2700

aaaaggttaa cataaaggct gtgttcaccc agtttgtact cgcaggtggt ttattaaaat 2760

catttgcaat atcaggtaat gagacgttca aaaccatttc atttaatacg ctaaaaaaag 2820

ataaaatgca aagccaaatt aaaatttggt tgtgtcgtaa attcgattgt gaataggatg 2880

tattcacatt tcaccctcca ataatgaggg cagacgtagt ttatagggtt aatgatacgc 2940

ttccctcttt taattgaacc ctgttacatt cattattcat tacacttcat aattaattcc 3000

tcctaaactt gattaaaaca ttttaccaca tataaactaa gttttaaatt cagtatttca 3060

tcacttatac aacaatatgg cccgtttgtt gaactactct ttaataaaat aatttttccg 3120

ttcccaattc cacattgcaa taatagaaaa tccatcttca tcggcttttt cgtcatcatc 3180

tgtatgaatc aaatcgcctt cttctgtgtc atcaaggttt aattttttat gtatttcttt 3240

taacaaacca ccataggaga ttaacctttt acggtgtaaa ccttcctcca aatcagacaa 3300

acgtttcaaa ttcttttctt catcatcggt cataaaatcc gtatccttta caggatattt 3360

tgcagtttcg tcaattgccg attgtatatc cgatttatat ttatttttcg gtcgaatcat 3420

ttgaactttt acatttggat catagtctaa tttcattgcc tttttccaaa attgaatcca 3480

ttgtttttga ttcacgtagt tttctgtatt cttaaaataa gttggttcca cacataccaa 3540

tacatgcatg tgctgattat aagaattatc tttattattt attgtcactt ccgttgcacg 3600

cataaaacca acaagatttt tattaatttt tttatattgc atcattcggc gaaatccttg 3660

agccatatct gacaaactct tatttaattc ttcgccatca taaacatttt taactgttaa 3720

tgtgagaaac aaccaacgaa ctgttggctt ttgtttaata acttcagcaa caaccttttg 3780

tgactgaatg ccatgtttca ttgctctcct ccagttgcac attggacaaa gcctggattt 3840

acaaaaccac actcgataca actttctttc gcctgtttca cgattttgtt tatactctaa 3900

tatttcagca caatctttta ctctttcagc ctttttaaat tcaagaatat gcagaagttc 3960

aaagtaatca acattagcga ttttcttttc tctccatggt ctcacttttc cactttttgt 4020

cttgtccact aaaacccttg atttttcatc tgaataaatg ctactattag gacacataat 4080

attaaaagaa acccccatct atttagttat ttgtttggtc acttataact ttaacagatg 4140

gggtttttct gtgcaaccaa ttttaagggt tttccaatac tttaaaacac atacatacca 4200

acacttcaac gcacctttca gcaactaaaa taaaaatgac gttatttcta tatgtatcaa 4260

gataagaaag aacaagttca aaaccatcaa aaaaagacac cttttcaggt gcttttttta 4320

ttttataaac tcattccctg atctcgactt cgttcttttt ttacctctcg gttatgagtt 4380

agttcaaatt cgttcttttt aggttctaaa tcgtgttttt cttggaattg tgctgtttta 4440

tcctttacct tgtctacaaa ccccttaaaa acgtttttaa aggcttttaa gcgtctgtac 4500

gttccttaag gaattattcc ttagtgcttt ctaggttaat gtcatgataa taatggtttc 4560

ttagacgtca ggtggcactt ttcggggaaa tgtccgcgga acccctattt gtttattttt 4620

ctaaatacat tcaaatatgt atccgctcat gagacaataa ccctgataaa tgcttcaata 4680

atattgaaaa aggaagagta tgagtattca acatttccgt gtcgccctta ttcccttttt 4740

tgcggcattt tgccttcctg tttttgctca cccagaaacg ctggtgaaag taaaagatgc 4800

tgaagatcag ttgggtgcac gagtgggtta catcgaactg gatctcaaca gcggtaagat 4860

ccttgagagt tttcgccccg aagaacgttt tccaatgatg agcactttta aagttctgct 4920

atgtggcgcg gtattatccc gtgttgacgc cgggcaagag caactcggtc gccgcataca 4980

ctattctcag aatgacttgg ttgagtactc accagtcaca gaaaagcatc ttacggatgg 5040

catgacagta agagaattat gcagtgctgc cataaccatg agtgataaca ctgcggccaa 5100

cttacttctg acaacgatcg gaggaccgaa ggagctaacc gcttttttgc acaacatggg 5160

ggatcatgta actcgccttg atcgttggga accggagctg aatgaagcca taccaaacga 5220

cgagcgtgac accacgatgc ctgcagcaat ggcaacaacg ttgcgcaaac tattaactgg 5280

cgaactactt actctagctt cccggcaaca attaatagac tggatggagg cggataaagt 5340

tgcaggacca cttctgcgct cggcccttcc ggctggctgg tttattgctg ataaatctgg 5400

agccggtgag cgtgggtctc gcggtatcat tgcagcactg gggccagatg gtaagccctc 5460

ccgtatcgta gttatctaca cgacggggag tcaggcaact atggatgaac gaaatagaca 5520

gatcgctgag ataggtgcct cactgattaa gcattggtaa ctgtcagacc aagtttactc 5580

atatatactt tagattgatt taaaacttca tttttaattt aaaaggatct aggtgaagat 5640

cctttttgat aatctcatga ccaaaatccc ttaacgtgag ttttcgttcc actgagcgtc 5700

agaccccgta ataagatgat cttcttgaga tcgttttggt ctgcgcgtaa tctcttgctc 5760

tgaaaacgaa aaaaccgcct tgcagggagg tttttcgaag gttctctgag ctaccaactc 5820

tttgaaccga ggtaactggc ttgcaggagc gcagtcacca aaacttgtcc tttcagttta 5880

gccttaaccg gcgcatgact tcaagactaa ctcctctaaa tcaattacca gtggctgctg 5940

ccagtggtgc ttttgcatgt ctttccgggt tggactcaag acgatagtta ccggataagg 6000

cgcagcggtc ggactgaacg gggggttcgt gcatacagtc cagcttggag cgaactgcct 6060

acccggaact gagtgtcagg cgtggaatga gacaaacgcg gccataacag cggaatgaca 6120

ccggtaaacc gaaaggcagg aacaggagag cgcacgaggg agccgccagg gggaaacgcc 6180

tggtatcttt atagtcctgt cgggtttcgc caccactgat ttgagcgtca gatttcgtga 6240

tgcttgtcag gggggcggag cctatggaaa aacggctttg ccc 6283

<210> 59
<211> 5884
<212> DNA
<213> Artificial sequence

<220>
<223> pCIP-CpB2

<400> 59
aagcttctag actatgcaca atatccttca ccaaatactc taattgatgc aggtgtaaag 60

ttttgtattc ttattctata tttatagaaa gattgtcctt tttctatata taatcttatt 120

tgtctagcag aatcagggtt ttgaccatac accatttgag aagctttaac atcatctccc 180

cataaccaat gatctacaac actatattct acttttaaat ctcttgaacc tgaagttgca 240

tattgtctaa agtttaataa tatattgtct gcataatatg taaatctagt agaatatgtt 300

tttggatcaa ctcccccaat tgattgtgat aattcatcat cccatgtaca aacttgagtt 360

ctaaatgatg gtgttaaaat atattttccc tcattctcaa tttttgttct ttccatgtca 420

gatactatta attccccatc tttagatgat acagtaaatt tcatatcata aattgcacca 480

ttcataaatt cagtttttac attaaatatt tctttattat caatcttttg tgaattaagt 540

atttcaaaag attttagttg ttggttagga tcatatttaa actcttctaa catctcttta 600

tacttttcat cactattcac tctttcatct tctgatacgt ttacaatcgt attaatatcg 660

tagtttttaa aagcattaag ataattctcc attacctttc tataagcgtc gatttccttt 720

gcacttgctt tcattggact tattgctcca acaataagaa aatatgaaaa cataaaaatt 780

acagtaaact ttgaaataat ttttttcata tgactccctc ctaattcaag tttatttaag 840

attcaatctt caaataatga aaatatattt taaatttttc tttttaacat aagataacct 900

cctttctatt ttgaatttag ttataatatt atgtattaat attatataaa tattatacag 960

tatatgacaa agaaaatctc tatataaata caaaaaatat atttttatca ataaaagtaa 1020

tttaatgtaa aattagattg tattttctga attcctgtta taaaaaaagg atcaattttg 1080
aactctctcc caaagttgat cccttaacga tttagaaatc cctttgagaa tgtttatata 1140
cattcaaggt aaccagccaa ctaatgacaa tgattcctga aaaaagtaat aacaaattac 1200
tatacagata agttgactga tcaacttcca taggtaacaa cctttgatca agtaagggta 1260
tggataataa accacctaca attgcaatac ctgttccctc tgataaaaag ctggtaaagt 1320
taagcaaact cattccagca ccagcttcct gctgtttcaa gctacttgaa acaattgttg 1380
atataactgt tttggtgaac gaaagcccac ctaaaacaaa tacgattata attgtcatga 1440
accatgatgt tgtttctaaa agaaaggaag cagttaaaaa gctaacagaa agaaatgtaa 1500
ctccgatgtt taacacgtat aaaggacctc ttctatcaac aagtatccca ccaatgtagc 1560
cgaaaataat gacactcatt gttccaggga aaataattac acttccgatt tcggcagtac 1620
ttagctggtg aacatctttc atcatataag gaaccataga gacaaaccct gctactgttc 1680
caaatataat tcccccacaa agaactccaa tcataaaagg tatatttttc cctaatccgg 1740
gatcaacaaa aggatctgtt actttcctga tatgttttac aaatatcagg aatgacagca 1800
cgctaacgat aagaaaagaa atgctatatg atgttgtaaa caacataaaa aatacaatgc 1860
ctacagacat tagtataatt cctttgatat caaaatgacc ttttatcctt acttctttct 1920
ttaataattt cataagaaac ggaacagtga taattgttat cataggaatg agtagaagat 1980
aggaccaatg aatataatgg gctatcattc caccaatcgc tggaccgact ccttctccca 2040
tggctactat cgatccaata agaccaaatg ctttacccct attttccttt ggaatatagc 2100
gcgcaactac aaccattacg agtgctggaa atgcagctgc accagcccct tgaataaaac 2160
gagccataat aagtaaggaa aagaaagaat ggccaacaaa cccaattacc gacccgaaac 2220
aatttattat aattccaaat aggagtaacc ttttgatgcc taattgatca gatagctttc 2280
catatacagc tgttccaatg gaaaaggtta acataaaggc tgtgttcacc cagtttgtac 2340
tcgcaggtgg tttattaaaa tcatttgcaa tatcaggtaa tgagacgttc aaaaccattt 2400
catttaatac gctaaaaaaa gataaaatgc aaagccaaat taaaatttgg ttgtgtcgta 2460
aattcgattg tgaataggat gtattcacat ttcaccctcc aataatgagg gcagacgtag 2520
tttatagggt taatgatacg cttccctctt ttaattgaac cctgttacat tcattattca 2580
ttacacttca taattaattc ctcctaaact tgattaaaac attttaccac atataaacta 2640
agttttaaat tcagtatttc atcacttata caacaatatg gcccgtttgt tgaactactc 2700
tttaataaaa taatttttcc gttcccaatt ccacattgca ataatagaaa atccatcttc 2760
atcggctttt tcgtcatcat ctgtatgaat caaatcgcct tcttctgtgt catcaaggtt 2820

taatttttta tgtatttctt ttaacaaacc accataggag attaaccttt tacggtgtaa 2880

accttcctcc aaatcagaca aacgtttcaa attcttttct tcatcatcgg tcataaaatc 2940

cgtatccttt acaggatatt ttgcagtttc gtcaattgcc gattgtatat ccgatttata 3000

tttatttttc ggtcgaatca tttgaacttt tacatttgga tcatagtcta atttcattgc 3060

ctttttccaa aattgaatcc attgtttttg attcacgtag ttttctgtat tcttaaaata 3120

agttggttcc acacatacca atacatgcat gtgctgatta taagaattat ctttattatt 3180

tattgtcact tccgttgcac gcataaaacc aacaagattt ttattaattt ttttatattg 3240

catcattcgg cgaaatcctt gagccatatc tgacaaactc ttatttaatt cttcgccatc 3300

ataaacattt ttaactgtta atgtgagaaa caaccaacga actgttggct tttgtttaat 3360

aacttcagca acaacctttt gtgactgaat gccatgtttc attgctctcc tccagttgca 3420

cattggacaa agcctggatt tacaaaacca cactcgatac aactttcttt cgcctgtttc 3480

acgattttgt ttatactcta atatttcagc acaatctttt actctttcag cctttttaaa 3540

ttcaagaata tgcagaagtt caaagtaatc aacattagcg attttctttt ctctccatgg 3600

tctcactttt ccactttttg tcttgtccac taaaaccctt gatttttcat ctgaataaat 3660

gctactatta ggacacataa tattaaaaga aacccccatc tatttagtta tttgtttggt 3720

cacttataac tttaacagat ggggtttttc tgtgcaacca attttaaggg ttttccaata 3780

ctttaaaaca catacatacc aacacttcaa cgcacctttc agcaactaaa ataaaaatga 3840

cgttatttct atatgtatca agataagaaa gaacaagttc aaaaccatca aaaaaagaca 3900

ccttttcagg tgcttttttt attttataaa ctcattccct gatctcgact tcgttctttt 3960

tttacctctc ggttatgagt tagttcaaat tcgttctttt taggttctaa atcgtgtttt 4020

tcttggaatt gtgctgtttt atcctttacc ttgtctacaa accccttaaa aacgttttta 4080

aaggctttta agcgtctgta cgttccttaa ggaattattc cttagtgctt tctaggttaa 4140

tgtcatgata ataatggttt cttagacgtc aggtggcact tttcggggaa atgtccgcgg 4200

aacccctatt tgtttatttt tctaaataca ttcaaatatg tatccgctca tgagacaata 4260

accctgataa atgcttcaat aatattgaaa aaggaagagt atgagtattc aacatttccg 4320

tgtcgccctt attccctttt ttgcggcatt ttgccttcct gtttttgctc acccagaaac 4380

gctggtgaaa gtaaaagatg ctgaagatca gttgggtgca cgagtgggtt acatcgaact 4440

ggatctcaac agcggtaaga tccttgagag ttttcgcccc gaagaacgtt ttccaatgat 4500

gagcactttt aaagttctgc tatgtggcgc ggtattatcc cgtgttgacg ccgggcaaga 4560

gcaactcggt cgccgcatac actattctca gaatgacttg gttgagtact caccagtcac 4620

agaaaagcat cttacggatg gcatgacagt aagagaatta tgcagtgctg ccataaccat 4680

gagtgataac actgcggcca acttacttct gacaacgatc ggaggaccga aggagctaac 4740

cgcttttttg cacaacatgg gggatcatgt aactcgcctt gatcgttggg aaccggagct 4800

gaatgaagcc ataccaaacg acgagcgtga caccacgatg cctgcagcaa tggcaacaac 4860

gttgcgcaaa ctattaactg gcgaactact tactctagct tcccggcaac aattaataga 4920

ctggatggag gcggataaag ttgcaggacc acttctgcgc tcggcccttc cggctggctg 4980

gtttattgct gataaatctg gagccggtga gcgtgggtct cgcggtatca ttgcagcact 5040

ggggccagat ggtaagccct cccgtatcgt agttatctac acgacgggga gtcaggcaac 5100

tatggatgaa cgaaatagac agatcgctga gataggtgcc tcactgatta agcattggta 5160

actgtcagac caagtttact catatatact ttagattgat ttaaaacttc atttttaatt 5220

taaaaggatc taggtgaaga tcctttttga taatctcatg accaaaatcc cttaacgtga 5280

gttttcgttc cactgagcgt cagacccctt aataagatga tcttcttgag atcgttttgg 5340

tctgcgcgta atctcttgct ctgaaaacga aaaaaccgcc ttgcagggag gtttttcgaa 5400

ggttctctga gctaccaact ctttgaaccg aggtaactgg cttgcaggag cgcagtcacc 5460

aaaacttgtc ctttcagttt agccttaacc ggcgcatgac ttcaagacta actcctctaa 5520

atcaattacc agtggctgct gccagtggtg cttttgcatg tctttccggg ttggactcaa 5580

gacgatagtt accggataag gcgcagcggt cggactgaac ggggggttcg tgcatacagt 5640

ccagcttgga gcgaactgcc tacccggaac tgagtgtcag gcgtggaatg agacaaacgc 5700

ggccataaca gcggaatgac accggtaaac cgaaaggcag gaacaggaga gcgcacgagg 5760

gagccgccag ggggaaacgc ctggtatctt tatagtcctg tcgggtttcg ccaccactga 5820

tttgagcgtc agatttcgtg atgcttgtca ggggggcgga gcctatggaa aaacggcttt 5880

gccc 5884

<210> 60
<211> 6106
<212> DNA
<213> Artificial sequence

<220>
<223> pCIP-BcSMase

<400> 60
aagcttctag actacttcat agaaatagtc gcctctactg gataatgatc agagtaatca 60

ttatacgtat atttttggaa ccacgatgta acagtccatt gtggagattt cggctgtaac 120

accttattct ctatatatga tgggttcgca tggtctttac ttgcaataat ataatctaaa 180

tactcagcag gactatcagg gaaattatat tttgcaatac tgtttgtcgt tgcgtcccaa 240
gtcgctgtat gtccagtata agatggtaca gaagcgttca atgttttaaa catagatgca 300
tactctgaat cattcttgtt ctctgcatta attttattta cgttcatatc accgccaatt 360
aacacatact cattatttgg tatattttta tttttaataa agtcttgaat ttcttttaat 420
tggtctgtac gtacagatgc aggtgaagtt tttccgcaca tactatcttc cgcttgcaaa 480
tgtgtaccaa tcacgtgaac gaagcgatca tttttcttaa ttttcgtgta tacaaatcct 540
ttattcgata aattatctgg cccgcatcct tttgcaaata catattgaat cttttcagcg 600
attggccatt tgctcacaat cgcaacgcca ccatcttcag gagttgaaga tgaatagttt 660
cctaacgttt tatcccattc acttccacta ctacgaccta atactgctgt ttgatttgga 720
tattctttct tcaaattccc taataaacga tctgaagcgc tattatcaaa cacttcattt 780
aatataacta catcttgatt ctttatataa tctgccgccc cgattaaatc agcacgctca 840
gtttgtcccc agttcggata taagtttgtt gatagcatat atacattatg cgtcatcact 900
tttaatgtat cattttgatt tgtagacact tctgcttgag ctgacgttcc gctatataaa 960
gctcctaaac caacacctaa gctaagtaca ccttttagca atttacattt cacgttccat 1020
acctccatca tatgactccc tcctaattca agtttattta agattcaatc ttcaaataat 1080
gaaaatatat tttaaatttt tctttttaac ataagataac ctcctttcta ttttgaattt 1140
agttataata ttatgtatta atattatata aatattatac agtatatgac aaagaaaatc 1200
tctatataaa tacaaaaaat atatttttat caataaaagt aatttaatgt aaaattagat 1260
tgtattttct gaattcctgt tataaaaaaa ggatcaattt tgaactctct cccaaagttg 1320
atcccttaac gatttagaaa tccctttgag aatgtttata tacattcaag gtaaccagcc 1380
aactaatgac aatgattcct gaaaaaagta ataacaaatt actatacaga taagttgact 1440
gatcaacttc cataggtaac aacctttgat caagtaaggg tatggataat aaaccaccta 1500
caattgcaat acctgttccc tctgataaaa agctggtaaa gttaagcaaa ctcattccag 1560
caccagcttc ctgctgtttc aagctacttg aaacaattgt tgatataact gttttggtga 1620
acgaaagccc acctaaaaca aatacgatta taattgtcat gaaccatgat gttgtttcta 1680
aaagaaagga agcagttaaa aagctaacag aaagaaatgt aactccgatg tttaacacgt 1740
ataaaggacc tcttctatca acaagtatcc caccaatgta gccgaaaata atgacactca 1800
ttgttccagg gaaaataatt acacttccga tttcggcagt acttagctgg tgaacatctt 1860
tcatcatata aggaaccata gagacaaacc ctgctactgt tccaaatata attcccccac 1920
aaagaactcc aatcataaaa ggtatatttt tccctaatcc gggatcaaca aaaggatctg 1980

ttactttcct gatatgtttt acaaatatca ggaatgacag cacgctaacg ataagaaaag 2040
aaatgctata tgatgttgta aacaacataa aaaatacaat gcctacagac attagtataa 2100
ttcctttgat atcaaaatga ccttttatcc ttacttcttt ctttaataat ttcataagaa 2160
acggaacagt gataattgtt atcataggaa tgagtagaag ataggaccaa tgaatataat 2220
gggctatcat tccaccaatc gctggaccga ctccttctcc catggctact atcgatccaa 2280
taagaccaaa tgctttaccc ctattttcct ttggaatata gcgcgcaact acaaccatta 2340
cgagtgctgg aaatgcagct gcaccagccc cttgaataaa acgagccata ataagtaagg 2400
aaaagaaaga atggccaaca aacccaatta ccgacccgaa acaatttatt ataattccaa 2460
ataggagtaa ccttttgatg cctaattgat cagatagctt tccatataca gctgttccaa 2520
tggaaaaggt taacataaag gctgtgttca cccagtttgt actcgcaggt ggtttattaa 2580
aatcatttgc aatatcaggt aatgagacgt tcaaaaccat ttcatttaat acgctaaaaa 2640
aagataaaat gcaaagccaa attaaaattt ggttgtgtcg taaattcgat tgtgaatagg 2700
atgtattcac atttcaccct ccaataatga gggcagacgt agtttatagg gttaatgata 2760
cgcttccctc ttttaattga accctgttac attcattatt cattacactt cataattaat 2820
tcctcctaaa cttgattaaa acattttacc acatataaac taagttttaa attcagtatt 2880
tcatcactta tacaacaata tggcccgttt gttgaactac tctttaataa aataattttt 2940
ccgttcccaa ttccacattg caataataga aaatccatct tcatcggctt tttcgtcatc 3000
atctgtatga atcaaatcgc cttcttctgt gtcatcaagg tttaattttt tatgtatttc 3060
ttttaacaaa ccaccatagg agattaacct tttacggtgt aaaccttcct ccaaatcaga 3120
caaacgtttc aaattctttt cttcatcatc ggtcataaaa tccgtatcct ttacaggata 3180
ttttgcagtt tcgtcaattg ccgattgtat atccgattta tatttatttt tcggtcgaat 3240
catttgaact tttacatttg gatcatagtc taatttcatt gcctttttcc aaaattgaat 3300
ccattgtttt tgattcacgt agttttctgt attcttaaaa taagttggtt ccacacatac 3360
caatacatgc atgtgctgat tataagaatt atctttatta tttattgtca cttccgttgc 3420
acgcataaaa ccaacaagat ttttattaat ttttttatat tgcatcattc ggcgaaatcc 3480
ttgagccata tctgacaaac tcttatttaa ttcttcgcca tcataaacat ttttaactgt 3540
taatgtgaga aacaaccaac gaactgttgg cttttgttta ataacttcag caacaacctt 3600
ttgtgactga atgccatgtt tcattgctct cctccagttg cacattggac aaagcctgga 3660
tttacaaaac cacactcgat acaactttct ttcgcctgtt tcacgatttt gtttatactc 3720
taatatttca gcacaatctt ttactctttc agccttttta aattcaagaa tatgcagaag 3780

ttcaaagtaa tcaacattag cgattttctt ttctctccat ggtctcactt ttccactttt 3840
tgtcttgtcc actaaaaccc ttgatttttc atctgaataa atgctactat taggacacat 3900
aatattaaaa gaaaccccca tctatttagt tatttgtttg gtcacttata actttaacag 3960
atggggtttt tctgtgcaac caattttaag ggttttccaa tactttaaaa cacatacata 4020
ccaacacttc aacgcacctt tcagcaacta aaataaaaat gacgttattt ctatatgtat 4080
caagataaga aagaacaagt tcaaaaccat caaaaaaaga caccttttca ggtgcttttt 4140
ttattttata aactcattcc ctgatctcga cttcgttctt tttttacctc tcggttatga 4200
gttagttcaa attcgttctt tttaggttct aaatcgtgtt tttcttggaa ttgtgctgtt 4260
ttatccttta ccttgtctac aaacccctta aaaacgtttt taaaggcttt taagcgtctg 4320
tacgttcctt aaggaattat tccttagtgc tttctaggtt aatgtcatga taataatggt 4380
ttcttagacg tcaggtggca cttttcgggg aaatgtccgc ggaaccccta tttgtttatt 4440
tttctaaata cattcaaata tgtatccgct catgagacaa taaccctgat aaatgcttca 4500
ataatattga aaaaggaaga gtatgagtat tcaacatttc cgtgtcgccc ttattccctt 4560
ttttgcggca ttttgccttc ctgtttttgc tcacccagaa acgctggtga aagtaaaaga 4620
tgctgaagat cagttgggtg cacgagtggg ttacatcgaa ctggatctca acagcggtaa 4680
gatccttgag agttttcgcc ccgaagaacg ttttccaatg atgagcactt ttaaagttct 4740
gctatgtggc gcggtattat cccgtgttga cgccgggcaa gagcaactcg gtcgccgcat 4800
acactattct cagaatgact tggttgagta ctcaccagtc acagaaaagc atcttacgga 4860
tggcatgaca gtaagagaat tatgcagtgc tgccataacc atgagtgata acactgcggc 4920
caacttactt ctgacaacga tcggaggacc gaaggagcta accgcttttt tgcacaacat 4980
gggggatcat gtaactcgcc ttgatcgttg ggaaccggag ctgaatgaag ccataccaaa 5040
cgacgagcgt gacaccacga tgcctgcagc aatggcaaca acgttgcgca aactattaac 5100
tggcgaacta cttactctag cttcccggca acaattaata gactggatgg aggcggataa 5160
agttgcagga ccacttctgc gctcggccct tccggctggc tggtttattg ctgataaatc 5220
tggagccggt gagcgtgggt ctcgcggtat cattgcagca ctggggccag atggtaagcc 5280
ctcccgtatc gtagttatct acacgacggg gagtcaggca actatggatg aacgaaatag 5340
acagatcgct gagataggtg cctcactgat taagcattgg taactgtcag accaagttta 5400
ctcatatata ctttagattg atttaaaact tcatttttaa tttaaaagga tctaggtgaa 5460
gatccttttt gataatctca tgaccaaaat cccttaacgt gagttttcgt tccactgagc 5520
gtcagacccc ttaataagat gatcttcttg agatcgtttt ggtctgcgcg taatctcttg 5580

ctctgaaaac gaaaaaaccg ccttgcaggg aggtttttcg aaggttctct gagctaccaa 5640

ctctttgaac cgaggtaact ggcttgcagg agcgcagtca ccaaaacttg tcctttcagt 5700

ttagccttaa ccggcgcatg acttcaagac taactcctct aaatcaatta ccagtggctg 5760

ctgccagtgg tgcttttgca tgtctttccg ggttggactc aagacgatag ttaccggata 5820

aggcgcagcg gtcggactga acggggggtt cgtgcataca gtccagcttg gagcgaactg 5880

cctacccgga actgagtgtc aggcgtggaa tgagacaaac gcggccataa cagcggaatg 5940

acaccggtaa accgaaaggc aggaacagga gagcgcacga gggagccgcc agggggaaac 6000

gcctggtatc tttatagtcc tgtcgggttt cgccaccact gatttgagcg tcagatttcg 6060

tgatgcttgt caggggggcg gagcctatgg aaaaacggct ttgccc 6106

<210> 61
<211> 6289
<212> DNA
<213> Artificial sequence

<220>
<223> pCIP-CbPLC

<400> 61
aagcttctag attatttatt atttatatag aatgttttgt tatctccaaa ccactcatta 60

atattagctt gatacttaac atctgaattt ataattactt ttacatattc aggtttccaa 120

tcatcttgaa ctccagcgcc tctttctttt cttatccatg ttttagctat atcttgaagt 180

gttaatttat tatcttttaa attaattcta taactatctt cttgattttt ttcaaaatca 240

ttacctggat tatcaagagt ccattcatac ttctttccat cttttgcttc aattccgaaa 300

tgaatagaat gatctgtacc tgctttatct tcatctgcag tttttattac tatcattatt 360

tcatttattt tagaattttc agttgtattt attgttcctg atacttcatt aaggaatctg 420

tatattattc cagcactacc tatttgaaca ttatgcatag tttcattagc tgctatttcc 480

caatctttcc aattatgatt catagtagca tgagcataat acatattctt agctttttta 540

gcccaacctc tagaatactc tgttatccaa ttatcaagat tagtattttg taatgtagtt 600

agataaaatt cctttacact attagttcct gctgaattta atttatatga atcttttctt 660

tcttcaacat aagtttcaaa tttcacatga cctgtactat ctacagctgt aacattagat 720

ggatgataag gagtattgaa atctccaaag taatgtaaac cttgtcctaa aagccatgtt 780

gcttgttcat aatttccttt tttccactca tctttagcta atgcaaataa ctttctaagt 840

tgagattctc ctgtttgact aattgcatat gatagatacc atttactatc tttagtaaaa 900

ttattatctg tatcaggatc ccaaaaatga tcttgatata atgcatatgc ctttggatca 960

taatctggat aagtagaacc atgttgtaat ttttttaaat tagattctaa aattttaaaa 1020

ttttctttta cacttaaagg tgatgtactt ataacatcat tttttaacat ttctacagct 1080

tgtgtaacta ttactgcatg agttcctgta ccatcaggtt ttccatccca tgcataactt 1140

ttatagcctg aaaataatgt gaatgataat actgcactac aaataaattt taatattttt 1200

ttcttattca tcatatgact ccctcctaat tcaagtttat ttaagattca atcttcaaat 1260

aatgaaaata tattttaaat ttttcttttt aacataagat aacctccttt ctattttgaa 1320

tttagttata atattatgta ttaatattat ataaatatta tacagtatat gacaaagaaa 1380

atctctatat aaatacaaaa aatatatttt tatcaataaa agtaatttaa tgtaaaatta 1440

gattgtattt tctgaattcc tgttataaaa aaaggatcaa ttttgaactc tctcccaaag 1500

ttgatccctt aacgatttag aaatcccttt gagaatgttt atatacattc aaggtaacca 1560

gccaactaat gacaatgatt cctgaaaaaa gtaataacaa attactatac agataagttg 1620

actgatcaac ttccataggt aacaaccttt gatcaagtaa gggtatggat aataaaccac 1680

ctacaattgc aatacctgtt ccctctgata aaaagctggt aaagttaagc aaactcattc 1740

cagcaccagc ttcctgctgt ttcaagctac ttgaaacaat tgttgatata actgttttgg 1800

tgaacgaaag cccacctaaa acaaatacga ttataattgt catgaaccat gatgttgttt 1860

ctaaaagaaa ggaagcagtt aaaaagctaa cagaaagaaa tgtaactccg atgtttaaca 1920

cgtataaagg acctcttcta tcaacaagta tcccaccaat gtagccgaaa ataatgacac 1980

tcattgttcc agggaaaata attacacttc cgatttcggc agtacttagc tggtgaacat 2040

ctttcatcat ataaggaacc atagagacaa accctgctac tgttccaaat ataattcccc 2100

cacaaagaac tccaatcata aaaggtatat ttttccctaa tccgggatca acaaaaggat 2160

ctgttacttt cctgatatgt tttacaaata tcaggaatga cagcacgcta acgataagaa 2220

aagaaatgct atatgatgtt gtaaacaaca taaaaaatac aatgcctaca gacattagta 2280

taattccttt gatatcaaaa tgacctttta tccttacttc tttctttaat aatttcataa 2340

gaaacggaac agtgataatt gttatcatag gaatgagtag aagataggac caatgaatat 2400

aatgggctat cattccacca atcgctggac cgactccttc tcccatggct actatcgatc 2460

caataagacc aaatgcttta cccctatttt cctttggaat atagcgcgca actacaacca 2520

ttacgagtgc tggaaatgca gctgcaccag ccccttgaat aaaacgagcc ataataagta 2580

aggaaaagaa agaatggcca acaaacccaa ttaccgaccc gaaacaattt attataattc 2640

caaataggag taaccttttg atgcctaatt gatcagatag ctttccatat acagctgttc 2700

caatggaaaa ggttaacata aaggctgtgt tcacccagtt tgtactcgca ggtggtttat 2760

taaaatcatt tgcaatatca ggtaatgaga cgttcaaaac catttcattt aatacgctaa 2820

aaaaagataa aatgcaaagc caaattaaaa tttggttgtg tcgtaaattc gattgtgaat 2880

aggatgtatt cacatttcac cctccaataa tgagggcaga cgtagtttat agggttaatg 2940

atacgcttcc ctcttttaat tgaaccctgt tacattcatt attcattaca cttcataatt 3000

aattcctcct aaacttgatt aaaacatttt accacatata aactaagttt taaattcagt 3060

atttcatcac ttatacaaca atatggcccg tttgttgaac tactctttaa taaaataatt 3120

tttccgttcc caattccaca ttgcaataat agaaaatcca tcttcatcgg ctttttcgtc 3180

atcatctgta tgaatcaaat cgccttcttc tgtgtcatca aggtttaatt ttttatgtat 3240

ttcttttaac aaaccaccat aggagattaa ccttttacgg tgtaaacctt cctccaaatc 3300

agacaaacgt ttcaaattct tttcttcatc atcggtcata aaatccgtat cctttacagg 3360

atattttgca gtttcgtcaa ttgccgattg tatatccgat ttatatttat ttttcggtcg 3420

aatcatttga acttttacat ttggatcata gtctaatttc attgcctttt tccaaaattg 3480

aatccattgt ttttgattca cgtagttttc tgtattctta aaataagttg gttccacaca 3540

taccaataca tgcatgtgct gattataaga attatcttta ttatttattg tcacttccgt 3600

tgcacgcata aaaccaacaa gatttttatt aattttttta tattgcatca ttcggcgaaa 3660

tccttgagcc atatctgaca aactcttatt taattcttcg ccatcataaa catttttaac 3720

tgttaatgtg agaaacaacc aacgaactgt tggcttttgt ttaataactt cagcaacaac 3780

cttttgtgac tgaatgccat gtttcattgc tctcctccag ttgcacattg gacaaagcct 3840

ggatttacaa aaccacactc gatacaactt tctttcgcct gtttcacgat tttgtttata 3900

ctctaatatt tcagcacaat cttttactct ttcagccttt ttaaattcaa gaatatgcag 3960

aagttcaaag taatcaacat tagcgatttt cttttctctc catggtctca cttttccact 4020

ttttgtcttg tccactaaaa cccttgattt ttcatctgaa taaatgctac tattaggaca 4080

cataatatta aaagaaaccc ccatctattt agttatttgt ttggtcactt ataactttaa 4140

cagatggggt ttttctgtgc aaccaatttt aagggttttc caatacttta aaacacatac 4200

ataccaacac ttcaacgcac ctttcagcaa ctaaaataaa aatgacgtta tttctatatg 4260

tatcaagata agaaagaaca agttcaaaac catcaaaaaa agacaccttt tcaggtgctt 4320

tttttatttt ataaactcat tccctgatct cgacttcgtt ctttttttac ctctcggtta 4380

tgagttagtt caaattcgtt ctttttaggt tctaaatcgt gtttttcttg gaattgtgct 4440

gttttatcct ttaccttgtc tacaaacccc ttaaaaacgt ttttaaaggc ttttaagcgt 4500

ctgtacgttc cttaaggaat tattccttag tgctttctag gttaatgtca tgataataat 4560

ggtttcttag acgtcaggtg gcacttttcg gggaaatgtc cgcggaaccc ctatttgttt 4620

atttttctaa atacattcaa atatgtatcc gctcatgaga caataaccct gataaatgct 4680

tcaataatat tgaaaaagga agagtatgag tattcaacat ttccgtgtcg cccttattcc 4740

cttttttgcg gcattttgcc ttcctgtttt tgctcaccca gaaacgctgg tgaaagtaaa 4800

agatgctgaa gatcagttgg gtgcacgagt gggttacatc gaactggatc tcaacagcgg 4860

taagatcctt gagagttttc gccccgaaga acgttttcca atgatgagca cttttaaagt 4920

tctgctatgt ggcgcggtat tatcccgtgt tgacgccggg caagagcaac tcggtcgccg 4980

catacactat tctcagaatg acttggttga gtactcacca gtcacagaaa agcatcttac 5040

ggatggcatg acagtaagag aattatgcag tgctgccata accatgagtg ataacactgc 5100

ggccaactta cttctgacaa cgatcggagg accgaaggag ctaaccgctt ttttgcacaa 5160

catgggggat catgtaactc gccttgatcg ttgggaaccg gagctgaatg aagccatacc 5220

aaacgacgag cgtgacacca cgatgcctgc agcaatggca acaacgttgc gcaaactatt 5280

aactggcgaa ctacttactc tagcttcccg gcaacaatta atagactgga tggaggcgga 5340

taaagttgca ggaccacttc tgcgctcggc ccttccggct ggctggttta ttgctgataa 5400

atctggagcc ggtgagcgtg ggtctcgcgg tatcattgca gcactggggc cagatggtaa 5460

gccctcccgt atcgtagtta tctacacgac ggggagtcag gcaactatgg atgaacgaaa 5520

tagacagatc gctgagatag gtgcctcact gattaagcat tggtaactgt cagaccaagt 5580

ttactcatat atactttaga ttgatttaaa acttcatttt taatttaaaa ggatctaggt 5640

gaagatcctt tttgataatc tcatgaccaa aatcccttaa cgtgagtttt cgttccactg 5700

agcgtcagac cccttaataa gatgatcttc ttgagatcgt tttggtctgc gcgtaatctc 5760

ttgctctgaa aacgaaaaaa ccgccttgca gggaggtttt tcgaaggttc tctgagctac 5820

caactctttg aaccgaggta actggcttgc aggagcgcag tcaccaaaac ttgtcctttc 5880

agtttagcct taaccggcgc atgacttcaa gactaactcc tctaaatcaa ttaccagtgg 5940

ctgctgccag tggtgctttt gcatgtcttt ccgggttgga ctcaagacga tagttaccgg 6000

ataaggcgca gcggtcggac tgaacggggg gttcgtgcat acagtccagc ttggagcgaa 6060

ctgcctaccc ggaactgagt gtcaggcgtg gaatgagaca aacgcggcca taacagcgga 6120

atgacaccgg taaaccgaaa ggcaggaaca ggagagcgca cgagggagcc gccaggggga 6180

aacgcctggt atctttatag tcctgtcggg tttcgccacc actgatttga gcgtcagatt 6240

tcgtgatgct tgtcaggggg gcggagccta tggaaaaacg gctttgccc 6289

<210> 62

<211>  5842
<212>  DNA
<213>  Artificial sequence

<220>
<223>  pCIP-CbC3

<400>  62
aagcttctag attatttagg attgatagct gtgcccatca ttgttgctgt aattattatt      60

tgtttaccat cagaagacaa tctcatatcg tctatatgat aagtactatg tctaggaagc     120

aacatttcaa gttgtcctgc aaaagcacta atagggtcaa tatatcctgc ctttgagcct     180

tttgctactt taaattttgt aataattggt cttcctgcaa attgagaaac attcattaat     240

gaagtactaa tatatccata ttcaagtcta tctttattta aaaacttagc tttagccttt     300

tcaaaagccg ttttattaat tgtaccattt gaattaagaa gagtgttttg aaattctgtt     360

cctaaataag cagggtcgtc gcctctaaat aacataatat tttcaggggt cttcatttta     420

ttaaaagatt tatctaaaag ttcaacttgt tttattaaat ttgaaggaaa tccattgata     480

actcccttat tttgtcttag ctttccattt atttcactag cgcttttagt atatgatact     540

atagcttctt tttctgattt gcttagtcca tactttttat actgagcatt accccaagct     600

tttgcttgat caatattagt aaactcctgg taagtatttg aataagcctt ttgattaatg     660

gaataagcat aacatttttg aggactttga atcatcccag atgttactgg agctattact     720

cctgctgaca aaactaaaca taaaattgat tttcttaaac ctttcatatg actccctcct     780

aattcaagtt tatttaagat tcaatcttca aataatgaaa atatatttta aatttttctt     840

tttaacataa gataacctcc tttctatttt gaatttagtt ataatattat gtattaatat     900

tatataaata ttatacagta tatgacaaag aaaatctcta tataaataca aaaaatatat     960

ttttatcaat aaaagtaatt taatgtaaaa ttagattgta ttttctgaat tcctgttata    1020

aaaaaaggat caattttgaa ctctctccca aagttgatcc cttaacgatt tagaaatccc    1080

tttgagaatg tttatataca ttcaaggtaa ccagccaact aatgacaatg attcctgaaa    1140

aaagtaataa caaattacta tacagataag ttgactgatc aacttccata ggtaacaacc    1200

tttgatcaag taagggtatg gataataaac cacctacaat tgcaatacct gttccctctg    1260

ataaaaagct ggtaaagtta agcaaactca ttccagcacc agcttcctgc tgtttcaagc    1320

tacttgaaac aattgttgat ataactgttt tggtgaacga aagcccacct aaaacaaata    1380

cgattataat tgtcatgaac catgatgttg tttctaaaag aaaggaagca gttaaaaagc    1440

taacagaaag aaatgtaact ccgatgttta acacgtataa aggacctctt ctatcaacaa    1500

gtatcccacc aatgtagccg aaaataatga cactcattgt tccagggaaa ataattacac    1560

ttccgatttc ggcagtactt agctggtgaa catctttcat catataagga accatagaga 1620
caaaccctgc tactgttcca aatataattc ccccacaaag aactccaatc ataaaaggta 1680
tatttttccc taatccggga tcaacaaaag gatctgttac tttcctgata tgttttacaa 1740
atatcaggaa tgacagcacg ctaacgataa gaaaagaaat gctatatgat gttgtaaaca 1800
acataaaaaa tacaatgcct acagacatta gtataattcc tttgatatca aaatgacctt 1860
ttatccttac ttctttcttt aataatttca taagaaacgg aacagtgata attgttatca 1920
taggaatgag tagaagatag gaccaatgaa tataatgggc tatcattcca ccaatcgctg 1980
gaccgactcc ttctcccatg gctactatcg atccaataag accaaatgct ttacccctat 2040
tttcctttgg aatatagcgc gcaactacaa ccattacgag tgctggaaat gcagctgcac 2100
cagccccttg aataaaacga gccataataa gtaaggaaaa gaaagaatgg ccaacaaacc 2160
caattaccga cccgaaacaa tttattataa ttccaaatag gagtaacctt ttgatgccta 2220
attgatcaga tagctttcca tatacagctg ttccaatgga aaaggttaac ataaaggctg 2280
tgttcaccca gtttgtactc gcaggtggtt tattaaaatc atttgcaata tcaggtaatg 2340
agacgttcaa aaccatttca tttaatacgc taaaaaaaga taaaatgcaa agccaaatta 2400
aaatttggtt gtgtcgtaaa ttcgattgtg aataggatgt attcacattt caccctccaa 2460
taatgagggc agacgtagtt tatagggtta atgatacgct tccctctttt aattgaaccc 2520
tgttacattc attattcatt acacttcata attaattcct cctaaacttg attaaaacat 2580
tttaccacat ataaactaag ttttaaattc agtatttcat cacttataca acaatatggc 2640
ccgtttgttg aactactctt taataaaata atttttccgt tcccaattcc acattgcaat 2700
aatagaaaat ccatcttcat cggctttttc gtcatcatct gtatgaatca aatcgccttc 2760
ttctgtgtca tcaaggttta attttttatg tatttctttt aacaaaccac cataggagat 2820
taacctttta cggtgtaaac cttcctccaa atcagacaaa cgtttcaaat tcttttcttc 2880
atcatcggtc ataaaatccg tatcctttac aggatatttt gcagtttcgt caattgccga 2940
ttgtatatcc gatttatatt tatttttcgg tcgaatcatt tgaactttta catttggatc 3000
atagtctaat ttcattgcct ttttccaaaa ttgaatccat tgtttttgat tcacgtagtt 3060
ttctgtattc ttaaaataag ttggttccac acataccaat acatgcatgt gctgattata 3120
agaattatct ttattattta ttgtcacttc cgttgcacgc ataaaaccaa caagattttt 3180
attaattttt ttatattgca tcattcggcg aaatccttga gccatatctg acaaactctt 3240
atttaattct tcgccatcat aaacattttt aactgttaat gtgagaaaca accaacgaac 3300
tgttggcttt tgtttaataa cttcagcaac aaccttttgt gactgaatgc catgtttcat 3360

tgctctcctc cagttgcaca ttggacaaag cctggattta caaaaccaca ctcgatacaa 3420
ctttctttcg cctgtttcac gattttgttt atactctaat atttcagcac aatcttttac 3480
tctttcagcc tttttaaatt caagaatatg cagaagttca aagtaatcaa cattagcgat 3540
tttcttttct ctccatggtc tcacttttcc actttttgtc ttgtccacta aaacccttga 3600
tttttcatct gaataaatgc tactattagg acacataata ttaaaagaaa cccccatcta 3660
tttagttatt tgtttggtca cttataactt taacagatgg ggtttttctg tgcaaccaat 3720
tttaagggtt ttccaatact ttaaaacaca tacataccaa cacttcaacg cacctttcag 3780
caactaaaat aaaaatgacg ttatttctat atgtatcaag ataagaaaga acaagttcaa 3840
aaccatcaaa aaaagacacc ttttcaggtg ctttttttat tttataaact cattccctga 3900
tctcgacttc gttctttttt tacctctcgg ttatgagtta gttcaaattc gttcttttta 3960
ggttctaaat cgtgtttttc ttggaattgt gctgttttat cctttacctt gtctacaaac 4020
cccttaaaaa cgtttttaaa ggcttttaag cgtctgtacg ttccttaagg aattattcct 4080
tagtgctttc taggttaatg tcatgataat aatggtttct tagacgtcag gtggcacttt 4140
tcggggaaat gtccgcggaa cccctatttg tttatttttc taaatacatt caaatatgta 4200
tccgctcatg agacaataac cctgataaat gcttcaataa tattgaaaaa ggaagagtat 4260
gagtattcaa catttccgtg tcgcccttat tccctttttt gcggcatttt gccttcctgt 4320
ttttgctcac ccagaaacgc tggtgaaagt aaaagatgct gaagatcagt tgggtgcacg 4380
agtgggttac atcgaactgg atctcaacag cggtaagatc cttgagagtt ttcgccccga 4440
agaacgtttt ccaatgatga gcacttttaa agttctgcta tgtggcgcgg tattatcccg 4500
tgttgacgcc gggcaagagc aactcggtcg ccgcatacac tattctcaga atgacttggt 4560
tgagtactca ccagtcacag aaaagcatct tacggatggc atgacagtaa gagaattatg 4620
cagtgctgcc ataaccatga gtgataacac tgcggccaac ttacttctga caacgatcgg 4680
aggaccgaag gagctaaccg cttttttgca caacatgggg gatcatgtaa ctcgccttga 4740
tcgttgggaa ccggagctga atgaagccat accaaacgac gagcgtgaca ccacgatgcc 4800
tgcagcaatg gcaacaacgt tgcgcaaact attaactggc gaactactta ctctagcttc 4860
ccggcaacaa ttaatagact ggatggaggc ggataaagtt gcaggaccac ttctgcgctc 4920
ggcccttccg gctggctggt ttattgctga taaatctgga gccggtgagc gtgggtctcg 4980
cggtatcatt gcagcactgg ggccagatgg taagccctcc cgtatcgtag ttatctacac 5040
gacggggagt caggcaacta tggatgaacg aaatagacag atcgctgaga taggtgcctc 5100
actgattaag cattggtaac tgtcagacca agtttactca tatatacttt agattgattt 5160

aaaacttcat ttttaattta aaaggatcta ggtgaagatc ctttttgata atctcatgac 5220

caaaatccct taacgtgagt tttcgttcca ctgagcgtca gaccccttaa taagatgatc 5280

ttcttgagat cgttttggtc tgcgcgtaat ctcttgctct gaaaacgaaa aaaccgcctt 5340

gcagggaggt ttttcgaagg ttctctgagc taccaactct ttgaaccgag gtaactggct 5400

tgcaggagcg cagtcaccaa aacttgtcct ttcagtttag ccttaaccgg cgcatgactt 5460

caagactaac tcctctaaat caattaccag tggctgctgc cagtggtgct tttgcatgtc 5520

tttccgggtt ggactcaaga cgatagttac cggataaggc gcagcggtcg gactgaacgg 5580

ggggttcgtg catacagtcc agcttggagc gaactgccta cccggaactg agtgtcaggc 5640

gtggaatgag acaaacgcgg ccataacagc ggaatgacac cggtaaaccg aaaggcagga 5700

acaggagagc gcacgaggga gccgccaggg ggaaacgcct ggtatcttta tagtcctgtc 5760

gggtttcgcc accactgatt tgagcgtcag atttcgtgat gcttgtcagg ggggcggagc 5820

ctatggaaaa acggctttgc cc 5842